(19) Europäisches Patentamt
European Patent Office
Office européen des brevets

(11) EP 1 090 297 B1

(12) **EUROPEAN PATENT SPECIFICATION**

(45) Date of publication and mention of the grant of the patent:
**06.08.2008 Bulletin 2008/32**

(21) Application number: **99929109.9**

(22) Date of filing: **24.06.1999**

(51) Int Cl.:
***G01N 33/543*** *(2006.01)* ***G01N 33/68*** *(2006.01)*

(86) International application number:
**PCT/DK1999/000362**

(87) International publication number:
**WO 1999/067642 (29.12.1999 Gazette 1999/52)**

# (54) METHOD OF DETECTING AN ANTIBODY IN A LIQUID SAMPLE

VERFAHREN ZUM NACHWEIS VON ANTIKÖRPERN IN FLÜSSIGEN PROBEN

PROCEDE DE DETECTION D'UN ANTICORPS DANS UN ECHANTILLON LIQUIDE

(84) Designated Contracting States:
**AT BE CH DE DK ES FI FR GB GR IE IT LI NL PT SE**

(30) Priority: **24.06.1998 DK 82198**

(43) Date of publication of application:
**11.04.2001 Bulletin 2001/15**

(73) Proprietor: **Alk-Abelló A/S**
**2970 Hørsholm (DK)**

(72) Inventors:
- **JOHANSEN, Niels**
  **DK-3450 Allered (DK)**
- **MOERKEBJERG, Rikke**
  **DK-2990 Niva (DK)**
- **BOEGESTRAND, Soeren**
  **DK-2980 Kokkedal (DK)**
- **BECK, Tine, Charlotte**
  **DK-3460 Birkeroed (DK)**
- **IPSEN, Hans-Henrik**
  **DK-3400 Hilleroed (DK)**

(74) Representative: **Christiansen, Ejvind et al**
**Zacco Denmark A/S**
**Hans Bekkevolds Allé 7**
**2900 Hellerup (DK)**

(56) References cited:

| | |
|---|---|
| **EP-A1- 0 291 180** | **EP-A2- 0 100 955** |
| **EP-A2- 0 119 613** | **EP-A2- 0 292 810** |
| **WO-A1-89/12231** | **WO-A1-90/08957** |
| **WO-A1-94/11734** | **WO-A1-94/29696** |
| **WO-A1-97/01758** | **WO-A1-98/16829** |
| **US-A- 4 444 879** | **US-A- 4 539 292** |
| **US-A- 5 643 733** | **US-A- 5 747 266** |

- **METHODS IN ENZYMOLOGY JEAN-LUC GUESDON ET AL: 'Magnetic Solid-Phase Enzyme Immunoassay for the Quantitation of Antigens and Antibodies: Application to Human Immunoglobulin E' vol. 73, 1981, pages 471 - 482, XP002921014**
- **JOURNAL OF IMMUNOLOGICAL METHODS MATHILDE POUSSIN ET AL: 'Capture-ELISA: a New Assay for the Detection of Immunoglobulin M Isotype Antibodies Using Chlamydia Trachomatis Antigen' vol. 204, 1997, pages 1 - 12, XP004061462**
- **EUR J CLIN CHEM CLIN BIOCHEM MARIO PLEBANI ET AL: 'Clinical Evaluation of a New Quantitative Method for Specific IgE Antibodies' vol. 34, 1996, BERLIN, NEW YORK, pages 579 - 584, XP002921015**
- **MASAHIRO SAKAGUCHI ET AL.: 'Measurement of antigen-specific mouse IgE by a fluorometric reverse (IgE-capture) ELISA' JOURNAL OF IMMUNOLOGICAL METHODS vol. 116, 1989, pages 181 - 187, XP002926656**
- **V. OLIVIERI ET AL.: 'Capture assay for specific IgE. An improved quantitative method' JOURNAL OF IMMUNOLOGICAL METHODS vol. 157, 1993, pages 65 - 72, XP002926655**
- **PAUL HERBRINK ET AL.: 'Interlaboratory Evaluation of Indirect Enzyme-Linked Immunosorbent Assay, Antibody Capture Enzymed-Linked Immunosorbent Assay and Immunoblotting for Detection of Immunoglobulin M Antibodies to Toxoplasmagondii' JOURNAL OF CLINICAL MICROBIOLOGY vol. 25, no. 1, January 1987, pages 100 - 105, XP002922982**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to a method of detecting a specific antibody in a liquid sample.

Prior art

WO 94/11734 describes a two-site immunoassay for an antibody using a chemiluminescent label and a biotin bound ligand, said method comprising the steps of (a) mixing the liquid sample with a ligand antigen, antibody or hapten bound to biotin or a functional derivative thereof, an antibody directed against the antibody to be detected bound to paramagnetic particles and a chemiluminescent acridinium compound bound to avidin, streptavidin or a functional derivative thereof to form a solid phase complex, (b) magnetically separating the solid phase from the liquid phase, (c) initiating a chemiluminescent reaction, if any, in the separated solid phase and (d) analysing the separated solid phase for the presence of a chemiluminescent phase, which is indicative of the presence of said antibody in the sample.
The prior art method is particularly suitable for measuring the concentration of specific immunoglobulins in body fluids, such as a specific immunoglobulin selected from the group of IgA, IgD, IgE, IgG, IgM and subclasses thereof.
The prior art method is also suitable for the detection and quantification of the total content of immunoglobulins in a class or subclass, such as IgA, IgD, IgE, IgG, IgM and subclasses thereof.
In a preferred embodiment of the prior art method the formation of the solid phase complex is effected in two steps, viz. a first step wherein the sample is mixed with the biotin bound ligand antigen, antibody or hapten and the antibody bound to paramagnetic particles so as to form a first solid phase complex, and a second step wherein the chemiluminescent acridinium compound is added to the first solid phase complex to form a second solid phase complex.
However, practical use of the prior art method has revealed the fact that in some applications of the method interference from other types of immunoglobulins and/or from other types of immunologically active serum components than the one to be measured occurs leading to errors in the results obtained.
The article "Capture assay for specific IgE", V. Olivieri et al, Journal of Immunological Methods, 157 (1993) 65-72 discloses an assay for the measurement of specific IgE in the serum of allergic patients using monoclonal anti-human IgE (coated to the wells of a microtiter plate) and biotinylated allergens in solution. In a single incubation IgE is bound to the solid phase through the Fc fragment and biotinylated allergens react with their specific IgE Fab regions. In a second step, streptavidin-horseradish peroxidase conjugate is added to reveal the amount of biotin fixed on the solid phase. The article studies the interference from high levels of non-specific IgE and from allergen-specific IgG. The assay is found to be unaffected by allergen-specific IgG.
WO 98/16829 discloses an assay, wherein an anti-immunoglobulin is coupled to a microtiter plate well, which is then washed. The test serum is then added to the well to capture all of total targeted immunoglobulin in the test sample. Then, the biological fluid sample is aspirated, and the microtiter plate is washed. The captured antibody on the microtiter plate is then exposed to biotinylated antigen, the plate is washed, and streptavidin/alkaline phosphatase conjugate is added, and the plate is washed again. The prior art assay may be used to monitor the effect of treatment on *H. Pylori* infection with standard antibiotic therapy.

Summary of the invention

A first object of the invention is to provide a method, which is capable of evaluating and/or predicting the effect of a Specific Allergy Vaccination (SAV).
This first object is obtained with the first aspect of the invention, which is defined in claim 1-6. According to this aspect the level, the nature and the temporal development of the interference between different types of immunologically active serum components, e.g. antibodies, are used as parameters for evaluating/predicting the effect of a Specific Allergy Vaccination treatment. Thus, it has surprisingly been found that the said parameters hold valuable information about the immunological status of a person as well as the response of a person to a selected treatment scheme.
A second object of the invention is to provide a method of evaluating the immunological status of a subject.
The second object of the invention is obtained with the second aspect of the invention, which is defined in claims 11-21. According to this aspect the level, the nature and the temporal development of the interference between different types of immunologically active serum components, e.g. antibodies, are used as a parameter for evaluating the immunological status of a subject, in particular evaluating/predicting the effect of allergy treatment, allergy vaccination treatment or Specific Allergy Vaccination treatment. Thus, it has surprisingly been found that the said parameter hold valuable information about the immunological status of a person as well as the response of a person to a selected treatment scheme.

First aspect of the invention

Specific allergy vaccination (SAV), formerly known as Specific Immunotheraphy or Hyposensitization, has been used for the treatment of Type 1 IgE mediated allergic disease since the beginning of this century.

The general benefits obtained through SAV are: a) reduction of allergic symptoms and medicine consumption, b) improved tolerance towards the allergens in the eyes, nose and lungs and c) reduced skin reactivity (early and late phase reactions).

The basic mechanism behind the improvement obtained by SAV is unknown, but a number of common features can be extracted from the numerous SAV studies performed in the last decades: 1) the amount of total IgE is unchanged during the treatment period, 2) the amount of allergen specific IgE increases transiently during updosing, then it falls back to the initial (pretreatment) level, 3) the epitope specificity and affinity of IgE remains unchanged, 4) allergen specific IgG, in particularly IgG4, raises sharply during SAV, 5) a new Th0/1 response is apparently initiated and 6) the Th2 response seem unchanged. There is no correlation between the effect induced by SAV and the onset of specific IgG.

SAV induces a new immune response which matures during the treatment period (Th0/1 T-cells are recruited, an allergen specific IgX (X may be A1, A2, G1, G2, G3, G4, M or D) is initiated). As the affinity (or amount/affinity) of the new antibody response, IgX, has matured, IgX may compete efficiently with IgE for the allergen(s), inhibiting the "normal" Th2 based allergic response characterised by the cross-linking of receptor bound IgE on the surface of mast-cells and basophils. Hence, clinical symptoms will gradually be reduced.

The present invention is based on the hypothesis that if one measures the amount of specific IgE in the presence and absence of competing compounds (IgX and/or any other interfering substance), a measure for the competitive capability of immune responses in the individual patient may be calculated and this measure would correlate with an appropriate effect parameter.

Most prior art "quantitative" IgE assays measure IgE in the absence of competing substances. The present invention describes the measurement of IgE in the presence and absence of any (serum originating) competing substance. Thus, the methods (cf. Figures 2a-c) measure IgE in the absence of competing agents, whereas the method defined in step (i'), and (y') of claims 1-6, respectively, measure IgE in the presence of competing agents.

It is believed that the mode of action of the method of the invention may be explained as follows: If SAV induces a response which competes with IgE for the binding of the allergen it should be possible to measure the effect of the treatment by comparing IgE determined by the above stated two methods. If the two methods produce the same result no interfering substance has been induced and there is no effect. Furthermore, if the measurement of the latter method (in the presence of competing substances) is lower than the measurement of the former method (in the absence of competing substances) a competing response has been mounted and there is an effect of the treatment.

In this aspect of the invention (Claims 1-6), two subassays are used in the method, viz. subassays ,wherein the reaction between sample antibody and allergen is effected in the absence (subassay (h') and (x') and presence (subassay (i') and (y') of the other sample constituents, respectively.

A preferred embodiment of the subassay (h') is characterized in that in step (a') components (i), (ii) and (iii) are mixed in one operation (Figure 3b).

A first alternative embodiment of the subassay (h') is characterized in that in step (a') components (i) and (ii) are mixed in a first operation and that component (iii) is added in a second operation (Figure 3c).

A second alternative subassay (h') is characterized in that in step (a') components (i) and (iii) are mixed in a first operation and that component (ii) is added in a second operation (Figure 3a).

A preferred embodiment of the first aspect of the invention is characterized in that step (ia') and (ya') is carried out by mixing components (i) and (ii), then adding component (iii), and finally adding component (iv), if added.

Another preferred embodiment of the first aspect of the invention is characterized in that step (ia') and (ya'), is carried out by mixing components (i), (ii) and (iii), and then adding component (iv), if added.

A further preferred embodiment is characterized in that the comparison of step (j') and (z') is carried out by calculating the ratio of the measurements of the said two steps. Alternatively, the comparison is carried out by calculating the difference between the two measurements.

Still a further embodiment of the invention is characterized in that the comparison of step (j') and (z') is carried out at a number of points in time at the start of and during the treatment period, and that any temporal change, which may be observed, is used as a basis for evaluating and/or predicting the effect of the treatment.

Second aspect of the invention

The second aspect of the invention is based on the same recognitions and hypothesis as the first aspect, the difference being that the second aspect is not limited to any specific immunoassay procedure. Also, according to the second aspect the assay may be used to predict the effect of all types of allergy treatment.

The interrelating of the two measurements obtained may be carried out by calculating the ratio of or the difference between the measurements.

The term "allergy treatment" means any treatment of allergy. The term "allergy vaccination treatment" means any vaccination treatment of allergy. The term "Specific Vaccination Treatment (SAV)" is described above.

Definitions

In the present invention the expressions "the antibody of the sample" and "sample antibody" may mean a specific immunoglobulin, preferably a specific immunoglobulin from the classes IgA, IgD, IgE, IgG, IgM and subclasses thereof. In general, the said antibody may be any blood serum or plasma component, which is capable of interfering specifically with the interaction between immunoglobulines and a ligand in the form of an antigen, an antibody or hapten, e.g. enzyme inhibitors and receptors.

The term "liquid sample" means any liquid or liquefied sample, including solutions, emulsions, dispersions and suspensions. The sample may be a biological fluid, such as blood, plasma, serum, urine, saliva and any other fluid, which is excreted, secreted or transported within a biological organism.

The expression "ligand in the form of an antigen, an antibody or a hapten" may be any immunologically active substance. "Antigen" may be an allergen, e.g. pollen from trees, grass, weeds etc., mould allergens, allergens from acarids (mites) and animals, such as cat, dog, horse, cattle and bird, allergens of stinging insects and inhaled allergens originating from insects, and food allergens; "antibody" may be a monoclonal or polyclonal antibody, including recombinant and fragmented antibodies; and "hapten" may be carbohydrate moieties or fragments thereof, enzyme inhibitors or drugs, e.g. penicillin or a derivative thereof.

The expression "labelled ligand" means any ligand comprising a labelled atom or part, e.g. a radioactive atom label.

The expressions "label compound" means any suitable label system conventionally used in immunoassays comprising luminescent labels, chemiluminescent labels, enzyme labels, radioactivity labels, fluorescent labels, and absorbance labels.

The term "carrier" means any solid support, which may be used in an 1mlnunoassay, e.g. a microtiter plate, a particle, a tube, a sponge of a polymer material (matrix) etc.

The term "solid particle" means any particulate matter, which can be suspended in a liquid, e.g. glass beads, metal, e.g. iron particles, particles of polymer material, etc.

The separation of the solid phase complex from the liquid phase may, depending on the type of solid particle used, be carried out by i.a. magnetic separation, filtration, sedimentation, centrifugation, chromatography, column chromatography.

The term "solid paramagnetic particle" means any paramagnetic particle, which may be dispersed or suspended in a liquid medium, e.g. "Biomag" particles (iron oxide particles coated with amine terminated groups) sold by Advanced Magnetics Inc., U.S.A., and "Dynabeads" (iron oxide covered with a polymer) sold by Dynal A.S., Norway.

The term "reactant antibody" means any antibody or other biospecific reagent capable of reacting with the sample antibody comprising monoclonal and polyclonal antibodies, including recombinant and fragmented antibodies, e.g. a monoclonal antibody, "MAb A 5697-1A3(920325) supplied by BioInvent International AB, Sweden, "Protein A" or "Protein G" supplied by Sigma Chemical Company, Saint Louis, USA.

The chemiluminescent compound is preferably an acridinium compound, such as N-hydroxy-succinimide dimethylacridiniumester (NHS-DMAE). Avidin/streptavidin and DMAE may be coupled according to the methods of Weeks et al., Clinical Chem., 29, 1474-1479 (1983). Other examples of chemiluminescent compounds suitable for use in the present invention are luminol, lucigenin and lophine.

In the following, the invention will be described in further detail with respect to the figures, wherein

Fig.1a-b are diagrammatic representations of two embodiments (Prior Art Method A and B) of the prior art assay disclosed in WO 94/11734.

Fig.2a-c are diagrammatic representations of three preferred assays suitable for use in the methods defined in claims 1-3 (submethods).

Fig.3a-c are diagrammatic representations of three assays.

Fig.4-6 show the chemiluminescence level as a function of the concentration of specific IgE for Prior Art Method B, Method 1 and Submethod 1, respectively.

Fig.7 shows the relative chemiluminescence level as a function of the content of interfering antibodies for Prior Art Method B, Method 1 and Submethod 1.

Fig.8-12 show the calculated chemiluminescence level relative to the expected level as a function of dilution factor for Prior Art Method B and Method 1 for four samples from patients subjected to Specific Allergy Vaccination and one reference sample (Fig.12).

Fig.13-14 show the relative response for patients subjected to Specific Allergy Vaccination and placebo treatment at 0, 6 and 12 months for Prior Art Method B and Method 1, respectively.

Fig.15 show the ratio of responses obtained with Prior Art Method B to responses obtained with Method 1.

Fig.16-18 show the relative IgE level at times 0, 6, 12 and 24 months for the three clinical effect groups Effect, No Effect and Doubtful, respectively.

Fig. 19 shows the ratio of responses obtained with Method 1 to responses obtained with Prior Art Method B as a function of time for various SAV treatment doses.

Fig. 20 shows the ratio of mean responses obtained with Method 1 to responses obtained with Prior Art Method B as a function of time for patients with clinical effect and patients without clinical effect, respectively.

Fig. 21 shows the ratio of responses obtained with Method 1 to responses obtained with Prior Art Method B as a function of time for individual patients with clinical effect.

Fig. 22 shows the ratio of responses obtained with Method 1 to responses obtained with Prior Art Method B as a function of time for individual patients with no effect.

Figure la shows the principle of one embodiment of the assay disclosed in WO 94/11734. In the assay the antibody to be detected (specific IgE) in the sample (i) is mixed with a reactant antibody bound to a paramagnetic particle (ii) to form a two-component complex, which is incubated, and then a biotinylated allergen (iii) is added to form a three-component complex, which is incubated, and then a chemiluminescent acridinium compound bound to avidin/streptavidin (iv) is added to form a four-component complex, which is washed, and the chemiluminescence of the washed complex is then measured.

Figure 1b shows another embodiment of the prior art assay, wherein components (i) and (iii) are mixed to form a two-component complex, and wherein components (ii) and (iv) are then added, and the resulting mixture is incubated to form a four-component complex, which is washed and subjected to chemiluminescence measurement.

In the assays of Figures 1a and 1b all constituents of the sample, including any cross-reacting IgE antibodies and non-IgE antibodies specific to the allergen, are present in the subsequent reactions leading to the formation of the four-component complex.

Figure 2a shows a method, wherein components (i) and (ii) are mixed and incubated to form a two-component complex, which is washed. Then component (iii) is added to form a three-component complex, after which component (iv) is added to form a four-component complex, which is washed and subjected to chemiluminescence measurement.

Figure 2b shows a method, which corresponds to that shown in Figure 2a except that components (iii) and (iv) are added in one operation.

Figure 2C shows a further method, which corresponds to that shown in Figure 2a except that the three-component complex formed is subjected to a washing step before the addition of component (iv). In the assays of Figures 2a, 2b and 2c the constituents of the sample, including any cross-reacting IgE antibodies and non-IgE antibodies specific to the allergen, are removed after reaction with component (ii) and are hence absent in the subsequent reaction steps leading to the formation of the four-component complex.

Figure 3a shows a preferred embodiment of the subassay (h') of claims 1-3, wherein components (i) and (iii) are mixed in a first operation to form a two-component complex, to which component (ii) is then added in a second operation, and the resulting mixture is incubated to form a three-component complex, which is washed before adding component (iv) to form a four-component complex, which is then washed before subjecting it to chemiluminescence measurement.

Figure 3b shows another preferred embodiment of subassay (h') of claims 1-3, wherein the components (i), (ii) and (iii) are added in one operation to form a three-component complex, which is then washed before adding component (iv) to form a four-component complex, which is then washed before subjecting it to chemiluminescence measurement.

Figure 3c shows a further preferred embodiment of subassay (h') of claims 1-3, wherein components (i) and (ii) are mixed to form a two-component complex, to which component (iii) is then added to form a three-component complex, which is washed before adding component (iv) to form a four-component complex, which is also washed before subjecting it to chemiluminescence measurement.

In the assays of Figures 3a, 3b and 3c the constituents of the sample, including any cross-reacting IgE antibodies and non-IgE antibodies specific to the allergen, are removed after reaction with components (ii) and (iii) and are hence absent in the subsequent reaction step leading to the formation of the four-component complex.

In the following the invention will be described in further detail with reference to the examples.

PREPARATION OF REAGENTS

<u>Biotinylated Dermatophagoides pteronyssinus</u>

Dermatophagoides pteronyssinus extract, (ALK-ABELLÓ A/S, Hørsholm, Denmark) is biotinylated in the molar ratio of 30:1. Biotin amidocaproate N-hydroxysuccinimide ester (Biotin-XX-NHS, Clontech, USA) is dissolved in Dimethylformamide (Merck) to a final concentration of 25 mg/ml. 55.4 μl of this solution of Biotin-XX-NHS is added to 1 ml of 2.44 mg/ml Dermatophagoides pteronyssinus in 0.1 M $NaHCO_3$, pH 8.5. The reagents are incubated for 15 minutes at 25°C in an "end over end" mixer. The biotinylated extract is purified from unbound biotin by size exclusion chromatography on a PD10-column (Pharmacia). The fraction containing the allergens is collected. The biotinylated Dermatophagoides pteronyssinus is diluted with Phosphate buffered saline, pH 7.2 (PBS), containing 0.1% human serum albumin (Sigma) and 0.09% $NaN_3$ (Merck).

Biotinylated Alternaria alternata

Alternaria alternata extract, (ALK-ABELLÓ A/S, Hørsholm, Denmark) is biotinylated in the molar ratio of 30:1. Biotin amidocaproate N-hydroxysuccinimide ester (Biotin-XX-NHS, Clontech, USA) is dissolved in Dimethylformamide (Merck) to a final concentration of 25 mg/ml. 38.6 μl of this solution of Biotin-XX-NHS is added to 1 ml of 1.7 mg/ml Alternaria alternata in 0.1 M $NaHCO_3$, pH 8.5. The reagents are incubated for 15 minutes at 25°C in an "end over end" mixer. The biotinylated extract is purified from unbound biotin by size exclusion chromatography on a PD10-column (Pharmacia). The fraction containing the allergens is collected. The biotinylated Alternaria alternata is diluted with Phosphate buffered saline, pH 7.2 (PBS), containing 0.1% human serum albumin (Sigma) and 0.09% $NaN_3$ (Merck).

Biotinylated Betula verrucosa (Silver birch)

Betula verrucosa pollen extract, (ALK-ABELLÓ A/S, Hørsholm, Denmark) is biotinylated in the molar ratio of 10:1. Biotin amidocaproate N-hydroxysuccinimide ester (Biotin-XX-NHS, Clontech, USA) is dissolved in Dimethylformamide (Merck) to a final concentration of 25 mg/ml. 33.5 μl of this solution of biotin-XX-NHS is added to 1 ml of 4.4 mg/ml Betula verrucosa in 0.1 M $NaHCO_3$, pH 8.5. The reagents are incubated for 15 minutes at 25°C in an "end over end" mixer. The biotinylated extract is purified from unbound biotin by size exclusion chromatography on a PD10-column (Pharmacia). The fraction containing the allergens is collected. The biotinylated Betula verrucosa is diluted with Phosphate buffered saline, pH 7.2 (PBS), containing 0.1% human serum albumin (Sigma) and 0.09% $NaN_3$ (Merck).

Preparation of streptavidin acridinium ester label

Streptavidin (Boehringer Mannheim) was conjugated with NSP-DMAE-NHS {2',6'-dimethyl-4'- (N-succinimidyloxycarbonyl)phenyl-10-(3-sulfopropyl)-acridinium-9-carboxylate} using a modified method of Weeks et al. (ref. 1).

Preparation of Streptavidin-acridinium ester label (lite reagent):

NSP-DMAE-NHS (Chiron Diagnostics, MA, USA) is dissolved in Dimethylformamide (Merck) to a final concentration of 1 mg/ml. 0.5 ml of this solution is added to 1.7 ml 5.88 mg/ml streptavidin in 0.1 M Sodium Phosphate buffer, 0.15 M NaCl, pH 8.5. The reagents are incubated for 30 minutes at 25°C with stirring. After incubation 0.4 ml 20 mg/ml 6-Amino-n-hexanoic acid (Sigma) is added. To remove unbound DMAE the solution is loaded onto a PD-10 column (Pharmacia, Uppsala, Sweden). The conjugated streptavidin is eluted with Phosphate buffered saline, pH 7.2 (PBS) and the fraction containing the conjugated streptavidin is collected. The conjugated streptavidin is diluted to a final concentration with Phosphate buffered saline, pH 7.2 (PBS) containing 0.1% HSA (Sigma), 1% Tween 20 (Merck) and 0.09% $NaN_3$.

Immobilization of antibody to paramagnetic particles:

6.5 g paramagnetic particles (Chiron Diagnostics, MA, USA) are washed 3 times in 650 ml 0.01 M acetate buffer pH 5.5 using magnetic separation. The particles are activated in 6.25% glutaraldehyde (Merck), 0.01 M acetate buffer pH 5.5 for 3 hours at 25°C. The particles are washed 6 times in 650 ml acetate buffer pH 5.5. The particles are coupled with 1625 mg monoclonal anti-IgE antibody (BioInvent International AB, Sweden) specific against the IgE Fc domain, for 16 hours at 25°C. The particles are washed twice in 650 ml 0.01 M phosphate buffer pH 7.4. Blocking of excess of active groups is performed with 1083 mg HSA (Sigma) dissolved in 0.01 M phosphate buffer pH 7.4 for 20 hours at 25°C. The particles are washed in 650 ml 0.01 M phosphate buffer pH 7.4 followed by 3 washes in 650 ml 1 M NaCl (Merck). The particles are washed 3 times in 0.01 M phosphate buffer pH 7.4 followed by a wash with 650 ml PBS pH 7.4, 0.1% HSA (Sigma). The particles are resuspended in 325 ml and heat treated for 16 hours at 50°C. The particles are washed 4 times in 650 ml 0.01 M phosphate pH7.4, 0.1% HSA. The particles are heat treated at 37°C for 7 days in 650 ml 0.01 M phosphate pH 7.4, 0.1% HSA with a buffer exchange on day 3 and day 5. The particles are diluted to a final concentration with Phosphate buffered saline, pH 7.2 (PBS) containing 0.1% HSA (Sigma) and 0.09% $NaN_3$.

Washing buffer for assays:

The washing buffer for assays is composed of 200 mM Potassium phosphate buffer, pH 7.4, 100 mM Potassium chloride, 0.1% Tween 20 and 0.09% $NaN_3$. All reagents are from Merck.

Example 1

Determination of specific antibody (specific IgE)

Determination of specific IgE antibodies against Betula verrucosa (Silver birch) allergen was performed by three different methods using the reagents described above in the working dilutions defined in each method. The three methods used were Prior Art Method B (Fig. 1b), Method 1 according to the invention (Fig. 2a, claim 2) and Submethod 1 according to the invention (Fig. 3a).

Prior Art Method B

This method is performed on Ciba Corning ACS:180 Benchtop Immunoassay Analyzer described in ref. 2. The analyzer is custom equipped with a 40 second time cycle software to give incubation times of two times the normal. 50 μl of sample and 50 μl of biotinylated Betula verrucosa allergen diluted 1:1500 are dispensed by the sample probe into the cuvette. When the cuvette after 12 minutes of incubation reaches reagent probe R2, 100 μl of paramagnetic particles diluted 1:20 and 200 μl of lite reagent diluted 1:10000 are dispensed simultaneously and the cuvette moves down the track. The magnets and the wash station are reached after additional 12 minutes and 40 seconds incubation. Wash with 750 μl deionized water is performed twice. After completion of the wash cycle the paramagnetic particles are resuspended in 300 μl 0.5 g/l $H_2O_2$ in 0.1 M $HNO_3$. The cuvette enters the luminometer chamber and in front of the photomultiplier 300 μl 25 mM NaOH solution is added and the photons of light emitted are measured and quantitated and expressed as relative light units (RLU). The amount of RLU is proportional to the amount of IgE in the sample. The time from sample dispension to first result is 30 minutes and a new result follows every 40 second. Results were expressed as RLU experiment/RLU background, where RLU background was the chemiluminescent reaction in the absence of IgE.

Method 1

This method is performed on a modified version of Ciba Corning ACS:180 Benchtop Immunoassay Analyzer described in ref. 2. 25 μl of sample is dispensed by the sample probe into the cuvette and immediately after this 100 μl of paramagnetic particles diluted 1:20 is dispensed by a fixed probe. After 8 minutes of incubation paramagnetic particles are magnetically separated and washed once with 1 ml of washing buffer. The paramagnetic particles are resuspended in 100 μl of washing buffer and 50 μl of biotinylated Betula verrucosa allergen diluted 1:1500 is added to the cuvette. After 10 minutes of incubation, 100 μl of lite reagent diluted 1:5000 is dispensed with a fixed probe and after additional 8 minutes of incubation the paramagnetic particles are magnetically separated and washed 3 times with 1 ml of washing buffer. After completion of the wash cycle the paramagnetic particles are resuspended in 300 μl 0.5 g/l $H_2O_2$ in 0.1 M $HNO_3$. The cuvette enters the luminometer chamber and in front of the photomultiplier 300 μl 25 mM NaOH solution is added and the photons of light emitted are measured and quantitated and expressed as relative light units (RLU). The amount of RLU is proportional to the amount of IgE in the sample. Results were expressed as RLU experiment/RLU background, where RLU background was the chemiluminescent reaction in the absence of IgE.

Submethod 1

This method is performed manually in 5 ml polystyrene test tubes (Sarstedt). 25 μl of sample is mixed with 50 μl of biotinylated Betula verrucosa allergen diluted 1:50. After 12 minutes of incubation at 37°C, 100 μl of paramagnetic particles diluted 1:20 are added and the mixture is incubated for 5 minutes and 30 seconds at 37°C before the paramagnetic particles are magnetically separated and washed once with 3 ml of washing buffer using a Magic Lite Multitube Washer (ALK-ABELLÓ A/S, Hørsholm, Denmark). The paramagnetic particles are resuspended in 200 μl of lite reagent diluted 1:10000 and incubated for 7 minutes and 30 seconds at 37°C before the paramagnetic particles are magnetically separated and washed 2 times with 3 ml of washing buffer using a Magic Lite Multitube Washer (ALK-ABELLÓ A/S, Hørsholm, Denmark). The paramagnetic particles are resuspended in 100 μl of deionized water and the chemiluminescent reaction is measured in a Magic Lite Analyzer II (ALK-ABELLO A/S, Hørsholm, Denmark) by adding first 300 μl 0.5 g/l $H_2O_2$ in 0.1 M $HNO_3$ and then 300 μl 25 mM NaOH solution. The photons of light emitted are measured and quantitated and expressed as relative light units (RLU). The amount of RLU is proportional to the amount of IgE in the sample. Results were expressed as RLU experiment/RLU background, where RLU background was the chemiluminescent reaction in the absence of IgE.

A serum pool of 5 birch pollen sensitive patients was serially diluted by a factor two from 140 SU/ml to 1.1 SU/ml to give 8 samples. These 8 samples were assayed together with a negative sample (background) and 8 samples from birch pollen sensitive individuals in each of the 3 above described methods, see Figures 4,5 and 6, respectively. It can be concluded that all assayed samples can be detected as positive using all three methods.

Example 2

Interference with specific antibodies from other classes (IgG)

Determination of specific IgE against Betula verrucosa (Silver birch) allergen was performed by the three different methods as described in Example 1 in the presence of varying amounts of competing specific antibodies. More specifically, a serum pool of 5 birch pollen sensitive patients was mixed in a 1:5 ratio with IgE negative serum containing different titers of rabbit polyclonal anti-Betula verrucosa antibody (ALK-ABELLÓ A/S, Hørsholm, Denmark). The control contained no rabbit antibody.

The results shown in Figure 7 are expressed as % recovery calculated as the response obtained with rabbit antibody in the sample relative to the control. Responses were given in Signal/Background. Recoveries less than 100% indicate that addition of competing antibody reduces the response while recoveries higher than 100% indicate that addition of competing antibody increases the response.

It can be concluded that Method 1 have less interference with competing antibodies from other classes than the simultaneous method. While addition of competing antibodies results in a significant positive interference in Submethod 1, especially with high amounts of interfering antibodies, interference seems to be eliminated completely in Method 1.

Example 3

Dilution curves with samples from patients undergoing Specific Allergy Vaccination (SAV)

Determination of specific IgE against Alternaria alternata (mould) allergen was performed by Prior Art Method B and Method 1 as described in Example 1 except that biotinylated Alternaria alternata allergen was used instead of biotinylated Betula verrucosa allergen. The working dilution of biotinylated Alternaria alternata allergen was 1:400 in both the Prior Art Method and Method 1.

Sera from four patients undergoing Specific Allergy Vaccination (SAV) were serially diluted by a factor two to give four samples prior to analysis in each of the two different methods. As a reference sample, a serum pool of 5 Alternaria alternata sensitive patients was used.

Figure 8-12 show results for samples from the four SAV patients and for the reference sample. The results shown in Figure 8 - 12 are the responses obtained with the different diluted sera relative to the expected responses. % Recovery is calculated as (Dilution factor)* (Observed response)/(Response of undiluted sample). Responses were given in Signal/Background. Recoveries less than 100% indicate that the responses of the diluted sera are lower than expected while recoveries higher than 100% indicate that the responses are higher than expected. Recoveries higher than 100% therefore indicate that the response obtained with the undiluted sample is an underestimate of the true value.

It appears from Figures 8 - 12 that for the SAV samples the Prior Art Method underestimate the true value of the undiluted SAV samples while Method 1 result in much more correct estimates. For a serum pool of patients not subjected to SAV both methods perform satisfactorily by giving much more accurate values.

It can be concluded that Method 1 containing 2 washing steps, can much more reliably estimate the amount of allergen specific IgE in serum samples from patients subjected to SAV than the Prior Art Method B.

Example 4

Monitoring of immune response during house dust mite SAV

Determination of specific IgE against Dermatophagoides pteronyssinus (House dust mite) allergen was performed by Prior Art Method B and Method 1 as described in Example 1 except that biotinylated Dermatophagoides pteronyssinus allergen was used instead of biotinylated Betula verrucosa allergen. The working dilution of biotinylated Dermatophagoides pteronyssinus allergen was 1:250 in both Prior Art Method B and Method 1.

Serum samples were obtained from patients involved in a clinical study. A total of 30 patients were given either placebo treatment (N=14) or SAV with a 1:1 mixture of Dermatophagoides pteronyssinus and Dermatophagoides farinae allergen (Alutard, ALK-ABELLÓ A/S, Hørsholm, Denmark) (N=16). Samples were drawn prior to treatment (t=0), after 6 months of treatment (t=6) and after 12 months of treatment (t=12) and all samples were analysed by the two different methods. Results were obtained as Relative response = (Signal-Background)/($Signal_{high}$-Background), where $Signal_{high}$ is the signal of a serum pool having a high content of the specific antibody to be detected. These results are shown in Figures 13 - 14. One additional set of data was generated by taking the ratio between results obtained in the Prior Art Method B and Method 1. These results are shown in Figure 15.

Conclusion:

Neither of the methods (Prior Art Method B or Method 1) used to determine serum specific IgE are capable of discriminating active and placebo treatment groups during SAV (Figures 13 - 14). However, the ratio of the results obtained from the Prior Art Method and the method of the invention (Ratio = IgE(Prior Art Method $B)_t$/IgE(Method $1)_t$) surprisingly discriminates active and placebo treatment groups 6 and 12 months after initiation of SAV (Figure 15). Further, it is likely that a ratio cut-off value of approximately 1 may predict if the individual patient belonged to the placebo or active treatment group (Figure 17, t=12) i.e. a SAV efficacy parameter.

Statistics:

Statistical analyses were performed by non-parametric tests (Mann-Whitney U-test). All two-sided stochastic probabilities less than 0.05 were considered significant. NS' signifies non-significant, otherwise the actual probabilities are stated in the Figures.

Example 5

Monitoring of immune response during house dust mite SAV

Determination of specific IgE antibodies against Dermatophagoides pteronyssinus (House dust mite) allergen was performed by two different methods using the reagents described above in the working dilutions defined in each method. The two methods used were Prior Art Method A (Fig.1a) and Method 1 according to the invention (Fig. 2a, claim 2)

Prior Art Method A

This method is performed on a modified version of Ciba Corning ACS:180 Benchtop Immunoassay Analyzer described in ref. 3. 25 µl of sample is dispensed by the sample probe into the cuvette and immediately after this 100 µl of paramagnetic particles diluted 1:20 is dispensed by a fixed probe. After 8 minutes of incubation paramagnetic particles are magnetically separated and not washed. The paramagnetic particles are resuspended in 100 µl of washing buffer and 50 µl of biotinylated Dermatophagoides pteronyssinus allergen diluted 1:250 is added to the cuvette. After 10 minutes of incubation, 100 µl of lite reagent diluted 1:5000 is dispensed with a fixed probe and after additional 8 minutes of incubation the paramagnetic particles are magnetically separated and washed 3 times with 1 ml of washing buffer. After completion of the wash cycle the paramagnetic particles are resuspended in 300 µl 0.5 g/ 1 $H_2O_2$ in 0.1 M $HNO_3$. The cuvette enters the luminometer chamber and in front of the photomultiplier 300 µl 25 mM NaOH solution is added and the photons of light emitted are measured and quantitated and expressed as relative light units (RLU). The amount of RLU is proportional to the amount of IgE in the sample. Results were expressed as RLU experiment/RLU background, where RLU background was the chemiluminescent reaction in the absence of IgE.

Method 1

This method is performed on a modified version of Ciba Corning ACS:180 Benchtop Immunoassay Analyzer described in ref. 2. 25 µl of sample is dispensed by the sample probe into the cuvette and immidiately after this 100 µl of paramagnetic particles diluted 1:20 is dispensed by a fixed probe. After 8 minutes of incubation paramagnetic particles are magnetically separated and washed once with 1 ml of washing buffer.
The paramagnetic particles are resuspended in 100 µl of washing buffer and 50 µl of biotinylated Dermatophagoides pteronyssinus allergen diluted 1:250 is added to the cuvette. After 10 minutes of incubation, 100 µl of lite reagent diluted 1:5000 is dispensed with a fixed probe and after additional 8 minutes of incubation the paramagnetic particles are magnetically separated and washed 3 times with 1 ml of washing buffer. After completion of the wash cycle the paramagnetic particles are resuspended in 300 µl 0.5 g/l $H_2O_2$ in 0.1 M $HNO_3$. The cuvette enters the luminometer chamber and in front of the photomultiplier 300 µl 25 mM NaOH solution is added and the photons of light emitted are measured and quantitated and expressed as relative light units (RLU). The amount of RLU is proportional to the amount of IgE in the sample. Results were expressed as RLU experiment/RLU background, where RLU background was the chemiluminescent reaction in the absence of IgE.

The present experimental work involve a total of 28 patients. 13 patients were not given any treatment, 7 patients were subjected to SAV treatment at a dosage level 100, and 8 patients were subjected to SAV treatment at a dosage level of 300. The SAV treatment consisted in administering to the patient dosages of Dermatophagoides pteronyssinus (House dust mite) allergen by subcutaneous injections initially during an up-dosing period of 21 weeks with weekly injections, and subsequently during a two-year period with dosages of 100 or 300 SQ-units administered every 6-8 weeks.

The study uses an effect parameter based upon skin prick test (SPT) and broncial provocation test (BPT). The clinical effect parameter utilised here has been obtained as follows: The relative skin indexes (SI(time=x)-SI(time=0)) were used to cluster the patients in two groups (effect = decrease in skin sensitivity and no effect = unchanged skin sensitivity). Likewise the relative provocation test (logBPT(Time=x)-logBPT(time=0)) were used to cluster the patients in two groups (effect = decreased broncial sensitivity and no effect = unchanged broncial sensitivity). The two measures were combined into one clinical effect parameter: clinical effect = effect in both measures, no clinical effect = no effect in both measures and doubtful clinical effect = effect in one of the measures and no effect in the other measure.

Figures 16-18 show the relative IgE level at times 0 (LT_S0_0), 6 (LT_S6_0), 12 (LT_S12_0) and 24 (LT_S24_0) months for the three clinical effect groups Effect, No Effect and Doubtful. Time = 0 is the pretreatment value. The curve indications having the format nn_iii signifies patient identification number (nn) and type of treatment (iii), wherein iii=0 means no treatment and iii = 100 or 300 means treatment at dosage level 100 and 300, respectively. The line at 0.14 is an arbitrary cut off value. The relative IgE level is defined as follows:

$$\log(\text{Response}(\text{Method 1})/\text{Response}(\text{Prior Art Method A}))_{t=x} - \log(\text{Response}(\text{Method 1})/\text{Response}(\text{Prior Art Method A}))_{t=0},$$

wherein t = time, x = 0, 6, 12 or 24 months and Response= Signal/Background.

As will appear from Figure 16, the response of Method 1 is higher than the response of Prior Art Method A for all of the patients that show a clinical effect, which means that a non IgE competing antibody response capable of reacting efficiently with the allergen has been initiated. It appears from Figure 17 that none of the patients in the clinical no effect group develop a competing antibody response. Finally, it appears from Figure 18 that one patient in the clinical doubtful group seem to develop a positive competing antibody response which are detected by only skin prick test. The test discriminates between treated and untreated patients since no patients from the control group (nn_0) is judged as if they raised a competing antibody response.

Conclusion

The ratio of IgE measured in the absence and presence of competing substances in the serum sample, i.e. measured by Method 1 and Prior Art Method A, respectively, seems to predict the effect of SAV. Furthermore, the in vitro measured effect is observable early in the treatment schedule. Although it is probable that the competing agent is an non IgE antibody - any serum substance reacting specifically with the allergens would be expected to behave similarly.

Example 6

Biotinylated Der p:

Der p 1 contains only one lysyl residue and the NH2-terminal aminoacid which are available for biotinylation. If one biotinylated a crude Der p extract using the "normal" labeling reagents (they target NH2-groups) the resulting labeled Der p extract tend to have biased sensitivity towards Der p 2 and other allergens.

A more balanced Der p reagent can be obtained as follows: crude Der p extract was biotinylated as described and a preparation of biotinylated Der p 1, labeled using biotin reagents capable of derivatizing tyrosine and histidine residues, was added. The resulting mixture was used in the following assays.

Biotinylation of Der p 1:

Der p 1 (ALK-Abelló A/S, Hørsholm, Denmark) was biotinylated with p-Diazobenzoyl Biocytin which was prepared from the stable precursor p-Aminobenzoyl Biocytin (Pierce, USA) according to the manufacturers instructions.

The resulting solution of p-Diazobenzoyl Biocytin had a theoretical concentration of 1.82 mg/ml (equivalent to 3.38 mM), assuming 100% efficiency of conversion from p-Aminobenzoyl Biocytin to p-Diazobenzoyl Biocytin. Der p 1 was biotinylated in a molar ratio of 17:1, assuming a molecular weight of Der p 1 of 25 kDa, by adding 343 μl of p-Diazobenzoyl Biocytin solution to 1 ml of 1.74 mg/ml Der p 1 in 0.1 M $NaHCO_3$, pH 8.5. The subsequent steps in the process was as previously described for the biotinylation with Biotin-XX-NHS ester.

Instrumentation:

The samples were analyzed using an ADVIA Centaur analyzer (Bayer Diagnostics). The amount of Der p specific IgE in each sample were determined utilizing two protocols: a two step method (T) involving washing procedures which remove interfering substances. (e.g. non IgE antibodies) before biotinylated allergens are added and a simultaneous protocol (S) which allow interfering substances (e.g. non IgE antibodies) to be present when the biotinylated allergens are added. Method T and Method S correspond to Method 1 and Prior Art Method A described in Example 5, except that the allergen was biotinylated as described above.

Materials and methods.

Biotinylated allergen reagent was made by making a 1:250 dilution of biotinylated Der p 1 and mixing 1 part of this reagent with 4 parts of normal Der p reagent prepared and diluted as previously described.

Serum samples from (N=48) Der p allergic patients receiving SAV with Der p, were obtained at 0, 0.5, 1, 2, 3, 6, 9, 12, 18 and 24 month after initiation of SAV. The patients were allocated in four treatment groups: controls (N=15), dose_10 (N=12), dose_100 (N=9) and dose_300 (N=11) and treated with Der p Alutard for 24 month with the dose indicated.

The clinical parameters SPT (Skin Prick Test), BPT (Bronchial Provocation Test) and CPT (Conjunctival Provocation Test) were measured before during and after SAV and a efficacy measure was constructed from the cluster analyzed clinical parameters. SAV was judged effective if two or three parameters was positive and noneffective if one or none of the parameters was positive.

IgE was measured in the serum samples according to the two protocols (T and S) and a baseline corrected in vitro efficacy parameter was calculated according to: LN(Ti/Si)-LN(T0/S0) where i is the time (>0<=24) and 0 is the pretreatment values.

Results:

There is a clear dose response relationship between LN(Ti/Si)-LN(TO/SO) and the dose given (Figure 19; Dose-response relation of the effect parameter) indicating that the parameter measures a dose dependent immunological change in the patients. Figure 20 (Mean effect parameter) illustrates the mean values obtained for the in vitro efficacy parameter for the patients with and without clinical effect and the in vitro parameter is shown for the individual patients in Figure 21 (Effect: individual patients) and Figure 22 (No effect: individual patients). All the patients with a positive clinical development show an increase of the in vitro parameter above the range of the control group from time 6 to 24 month and all these patients were receiving an active Der p SAV. Four patients (14%) with no apparent clinical effect are judged to have an effect by the in vitro effect parameter.

Conclusion:

The in vitro effect parameter seems to correlate well with the clinical assessment of the patient status, and the in vitro parameter is predictive of the outcome of SAV after 6 month of SAV whereas the clinical effects normally are obvious after 12 or 24 month.

REFERENCES

1) Ian Weeks, Iraj Beheshti, Frank McCapra, Anthony K. Campbell and J. Stuart Woodhead, Clinical Chemistry, 1983, 29, 1474 - 1479 (1983).

2) J. Boland, G. Carey, E. Krodel and M. Kwiatkowski, Clinical Chemistry, 1990, 36, 1598 - 1602.

## Claims

1. A method of evaluating and/or predicting the effect of a Specific Allergy Vaccination treatment comprising the steps of (h') determining the content of an antibody in a liquid sample using the following assay:

    (a') providing a mixture of a liquid phase and a three-component solid phase complex composed of (i) the antibody of the sample, (ii) a reactant antibody directed against the sample antibody, the reactant antibody being bound to a solid particle, and (iii) a ligand in the form of an antigen, an antibody or a hapten,
    (b') separating the three-component solid phase complex from the liquid phase,
    (c') washing the separated three-component solid phase complex to remove non-complex bound compounds,
    (d') adding to the three-component solid phase complex a solution of (iv) a label compound to form a four-component complex,
    (e') separating the four-component solid phase complex from the solution,
    (f') washing the separated four-component solid phase complex to remove non-complex bound compound (iv),
    (g') performing a detection/measurement of the washed labelled four-component complex,

    (i') determining the content of the said antibody using the following assay:

    (ia') providing a mixture of a liquid phase and a four-component solid phase complex composed of (i) the antibody of the sample, (ii) a reactant antibody directed against the sample antibody, the reactant antibody being bound to a solid particle, (iii) a ligand in the form of an antigen, an antibody or a hapten, and (iv) a label compound, to form a four-component solid phase complex,
    (ib') separating the four-component solid phase complex from the liquid phase,
    (ic') washing the separated four-component solid phase complex to remove non-complex bound compounds,
    (id') performing a detection/measurement of the washed labelled four-component complex,

    (j') comparing the measurements obtained in step (h') and step (i') and using the comparison to evaluate and/or predict the effect of the Specific Allergy Vaccination treatment.

2. A method according to claim 1,
    wherein in step (a') and in step (ia') the ligand is bound to biotin or a functional derivative thereof, and
    wherein in step (d') and in step (ia') the label compound is a chemiluminescent compound covalently bound to avidin, streptavidin or a functional derivative thereof, and
    wherein in step (g') and in step (id') the detection/measurement is carried out by initiating a chemiluminescent reaction in the washed four-component solid phase complex and detecting/measuring the resulting chemiluminescence, if any.

3. A method according to claim 2,
    wherein in step (a') and in step (ia') the solid particle is a solid paramagnetic particle, and
    wherein in step (b') and in step (ib') the separation of the solid phase complex from the liquid phase is performed magnetically.

4. A method of evaluating and/or predicting the effect of a Specific Allergy Vaccination treatment comprising the steps of (x') determining the content of an antibody in a liquid sample using the following method:

    (o') providing a mixture of a liquid phase and a two-component solid phase complex composed of (i) the antibody of the sample, and (ii) a reactant antibody directed against the sample antibody, the reactant antibody being bound to a solid particle,
    (p') separating the two-component solid phase complex from the liquid phase,
    (q') washing the separated two-component solid phase complex to remove non-complex bound compounds,
    (r') adding to the washed two-component solid phase complex a solution of (iii) a ligand in the form of an antigen, an antibody or a hapten, which is optionally labelled, to form a three-component solid phase complex,
    (s') optionally adding to the three-component solid phase complex a solution of (iv) a label compound to form a four-component solid phase complex,
    (t') separating the three- or four-component solid phase complex obtained in step (r') or (s'), respectively, from the solution,
    (u') washing the separated three- or four-component solid phase complex to remove non-complex bound compounds,

(v') performing a detection/measurement of the washed labelled three- or four-component complex,

(y') determining the content of the said antibody using the following assay:

(ya') providing a mixture of a liquid phase and a three-component solid phase complex composed of (i) the antibody of the sample, (ii) a reactant antibody directed against the sample antibody, the reactant antibody being bound to a solid particle, (iii) a ligand in the form of an antigen, an antibody or a hapten, which is labelled or bound to (iv) a label compound, to form a multi-component solid phase complex,
(yb') separating the multi-component solid phase complex from the liquid phase,
(yc') washing the separated multi-component solid phase to remove non-complex bound compounds,
(yd') performing a detection/measurement of the washed labelled multi-component complex.

(z') comparing the measurements obtained in step (x') and step (y') and using the comparison to evaluate and/or predict the effect of the Specific Allergy Vaccination treatment.

**5.** A method according to claim 4,
wherein in step (r') and in step (ya') the ligand is bound to biotin or a functional derivative thereof, and
wherein step (s') is mandatory and wherein in step (s') and in step (ya') the label compound is a chemiluminescent compound covalently bound to avidin, streptavidin or a functional derivative thereof, and
wherein in step (v') and in step (yd') the detection/measurement is carried out by initiating a chemiluminescent reaction in the washed four-component solid phase complex and detecting/measuring the resulting chemiluminescence, if any.

**6.** A method according to claim 5,
wherein in step (o') and in step (ya') the solid particle is a solid paramagnetic particle, and
wherein in step (p'), in step (t') and in step (yb') the separation of the solid phase complex from the liquid phase is performed magnetically.

**7.** A method according to claim 1, 2, 3, 4, 5 or 6, wherein step (ia') and (ya') respectively, is carried out by mixing components and (ii), then adding component (iii), and finally adding component (iv), if added.

**8.** A method according to claim 1, 2, 3, 4, 5 or 6, wherein step (ia') and (ya') respectively, is carried out by mixing components (i), (ii) and (iii), and then adding component (iv), if added.

**9.** A method according to claim 1, 2, 3, 4, 5 or 6, wherein the comparison of step (j') and (z'), respectively, is carried out by calculating the ratio of the measurements obtained in the two said steps.

**10.** A method according to any of claim 1, 2, 3, 4, 5 or 6, wherein the comparison of step (j') and (z'), respectively, is carried out at a number of points in time at the start of and during the treatment period, and that any temporal change, which may be observed, is used as a basis for evaluating and/or predicting the effect of the treatment.

**11.** A method of evaluating the immunological status of an allergic subject comprising the steps of

A) determining the content of an antibody in a liquid sample from the subject using the following assay protocol (assay A):
(Aa) mixing (i) the antibody of the sample, (ii) an antibody directed against the Fc region of the sample antibody, the reactant antibody being bound to a solid carrier and (iii) a ligand in the form of an antigen, an antibody or a hapten, the ligand being directed to the Fab region of the sample antibody, to form a mixture of a three-component solid phase complex and a liquid phase,
(Ab) contacting the three-component complex with (iv) a label compound to form a mixture of a four-component complex and a liquid phase,
(Ac) washing the four-component solid phase to remove non-complex bound compounds,
(Ad) performing a detection/measurement of the washed labelled four-component complex to obtain a measurement A;
B) determining the content of the said antibody in the said sample using the following assay protocol (assay B):
(Ba) mixing (i) the antibody of the sample, and (ii) a reactant antibody directed against the Fc region of the sample antibody, the reactant antibody being bound to a. solid carrier, to form a mixture of a two-component solid phase complex and a liquid phase,

(Bb) washing the two-component solid phase complex to remove non-complex bound compounds,
(Bc) contacting the washed two-component solid phase complex with (iii) a ligand in the form of an antigen, an antibody or a hapten, the ligand being bound to the Fab region of the sample antibody, and (iv) a label compound, to form a mixture of a four-component solid phase complex and a liquid phase,
(Bd) washing the four-component solid phase complex to remove non-complex bound compounds,
(Be) performing a detection/measurement of the washed labelled four-component complex to obtain a measurement B; and
(E) interrelating measurements A and B to express the interference and using the interference as a parameter for evaluating the immunological status of the subject.

**12.** A method according to claim 11, wherein step Ab is effected by adding said (iv) label compound to said mixture of a three-component solid phase complex and a liquid phase in step Aa.

**13.** A method according to claim 11, wherein step Ab is effected by washing the three-component complex obtained in step Aa to remove non-complex bound compounds and by subsequently adding said (iv) label compound to the washed three-component complex.

**14.** A method according to any of claims 11-13, wherein step Bc is effected by simultaneous incubation of said (iii) ligand and said (iv) label compound with the two-component complex.

**15.** A method according to any of claims 11-13, wherein initially said (iii) ligand is added to the two-component complex to form a three-component complex, which is then washed to remove non-complex bound (iii) ligand, and then said (iv) label compound is added to form a four-component complex.

**16.** A method according to any of claims 11-15, wherein the label compound is a luminescent label, a chemiluminescent label, an enzyme label, a radioactivity label, a fluorescent label or an absorbance label.

**17.** A method according to any of claims 11-16, wherein the (iii) ligand is biotinylated.

**18.** A method according to claim 17, wherein the (iv) label compound is a chemiluminescent compound covalently bound to avidin, streptavidin or a functional derivative thereof.

**19.** A method of evaluating the immunological status of an allergic subject comprising the steps of

C) determining the content of an antibody in a liquid sample from the subject using the following assay protocol (assay C):
(Ca) mixing (i) the antibody of the sample, (ii) an antibody directed against the Fc region of the sample antibody, the reactant antibody being bound to a solid carrier and (iii) a labelled ligand in the form of an antigen, an antibody or a hapten, the ligand being directed to the Fab region of the sample antibody, to form a mixture of a three-component solid phase complex and a liquid phase,
(Cb) washing the three-component solid phase to remove non-complex bound compounds,
(Cc) performing a detection/measurement of the washed labelled three-component complex to obtain a measurement C;
D) determining the content of the said antibody using the following assay protocol (assay D):
(Da) mixing (i) the antibody of the sample, and (ii) a reactant antibody directed against the Fc region of the sample antibody, the reactant antibody being bound to a solid carrier, to form a mixture of a two-component solid phase complex and a liquid phase,
(Db) washing the two-component solid phase complex to remove non-complex bound compounds,
(Dc) contacting the washed two-component solid phase complex with a (iii) a labelled ligand in the form of an antigen, an antibody or a hapten, the ligand being bound to the Fab region of the sample antibody, to form a mixture of a three-component solid phase complex and a liquid phase,
(Dd) washing the three-component solid phase complex to remove non-complex bound compounds,
(De) performing a detection/measurement of the washed labelled three-component complex to obtain a measurement D; and
(E) interrelating measurements C and D to express the interference and using the interference as a parameter for evaluating the immunological status of the subject.

**20.** A method according to claim 19, wherein the labelled ligand is labelled by a radioactive atom.

21. A method according to any of claims 11-20, wherein the subject to be evaluated is undergoing allergy treatment, allergy vaccination treatment or Specific Allergy Vaccination (SAV) treatment.

**Patentansprüche**

1. Methode zum Auswerten und/oder Vorhersagen der Wirkung einer spezifischen Allergie-Vakzinationsbehandlung, umfassend die folgenden Schritte
(h') Bestimmen des Gehalts an einem Antikörper in einer Flüssigkeitsprobe unter Anwendung des folgenden Assays:

(a') Bereitstellen eines Gemischs aus einer Flüssigphase und einem dreikomponentigen Festphasen-Komplex, zusammengesetzt aus (i) dem Antikörper aus der Probe, (ii) einem Reaktant-Antikörper, der gegen den Proben-Antikörper gerichtet ist, wobei der Reaktant-Antikörper an einen festen Partikel gebunden ist, und (iii) einem Liganden in der Form eines Antigens, eines Antikörpers oder eines Haptens,
(b') Trennen des dreikomponentigen Festphasen-Komplexes von der Flüssigphase,
(c') Waschen des abgetrennten dreikomponentigen Festphasen-Komplexes; um nicht-Komplex-gebundene Verbindungen zu entfernen,
(d') Zugeben zu dem dreikomponentigen Festphasen-Komplex einer Lösung von (iv) einer Markierungsverbindung, um einen vierkomponentigen Komplex zu bilden,
(e') Trennen des vierkomponentigen Festphasen-Komplexes von der Flüssigphase,
(f') Waschen des abgetrennten vierkomponentigen Festphasen-Komplexes, um nicht-Komplex-gebundene Verbindung (iv) zu entfernen,
(g') Durchführen einer Detektion/Messung des gewaschenen markierten vierkomponentigen Komplexes,

(i') Bestimmen des Gehalts an dem Antikörper unter Anwendung des folgenden Assays:

(ia') Bereitstellen eines Gemischs aus einer Flüssigphase und einem vierkomponentigen Festphasen-Komplex, zusammengesetzt aus (i) dem Antikörper aus der Probe, (ii) einem Reaktant-Antikörper, der gegen den Proben-Antikörper gerichtet ist, wobei der Reaktant-Antikörper an einen festen Partikel gebunden ist, und (iii) einem Liganden in der Form eines Antigens, eines Antikörpers oder eines Haptens, und (iv) einer Markierungsverbindung, um einen vierkomponentigen Festphasen-Komplex zu bilden,
(ib') Trennen des vierkomponentigen Festphasen-Komplexes von der Flüssigphase,
(ic') Waschen des abgetrennten vierkomponentigen Festphasen-Komplexes, um nicht-Komplex-gebundene Verbindungen zu entfernen,
(id') Durchführen einer Detektion/Messung des gewaschenen markierten vierkomponentigen Komplexes,

(j') Vergleichen der Messungen, die in Schritt (h') und in Schritt (i') erhalten wurden, und Verwenden des Vergleichs zur Auswertung und/oder Vorhersage der Wirkung der spezifischen Allergie-Vakzinationsbehandlung.

2. Methode nach Anspruch 1,
wobei in Schritt (a') und in Schritt (ia') der Ligand an Biotin oder ein funktionelles Derivat davon gebunden ist, und wobei in Schritt (d') und in Schritt (ia') die Markierungsverbindung eine chemilumineszierende Verbindung ist, die an Avidin, Streptavidin oder ein funktionelles Derivat davon kovalent gebunden ist, und
wobei in Schritt (g') und in Schritt (id') die Detektion/Messung durch Initiieren einer chemilumineszierenden Reaktion in dem gewaschenen vierkomponentigen Festphasen-Komplex und Detektieren/Messen der resultierenden Chemilumineszenz, sofern vorhanden, durchgeführt wird.

3. Methode nach Anspruch 2,
wobei in Schritt (a') und in Schritt (ia') der feste Partikel ein fester paramagnetischer Partikel ist, und
wobei in Schritt (b') und in Schritt (ib') die Trennung des Festphasen-Komplexes von der Flüssigphase magnetisch vorgenommen wird.

4. Methode zum Auswerten und/oder Vorhersagen der Wirkung einer spezifischen Allergie-Vakzinationsbehandlung, umfassend die folgenden Schritte
(x') Bestimmen des Gehalts an einem Antikörper in einer Flüssigkeitsprobe unter Anwendung der folgenden Methode:

(o') Bereitstellen eines Gemischs aus einer Flüssigphase und einem zweikomponentigen Festphasen-Komplex, zusammengesetzt aus (i) dem Antikörper aus der Probe, und (ii) einem Reaktant-Antikörper, der gegen den Proben-Antikörper gerichtet ist, wobei der Reaktant-Antikörper an einen festen Partikel gebunden ist,
(p') Trennen des zweikomponentigen Festphasen-Komplexes von der Flüssigphase,
(q') Waschen des abgetrennten zweikomponentigen Festphasen-Komplexes, um nicht-Komplex-gebundene Verbindungen zu entfernen,
(r') Zugeben zu dem gewaschenen zweikomponentigen Festphasen-Komplex einer Lösung von (iii) einem Liganden in der Form eines Antigens, eines Antikörpers oder eines Haptens, welcher/s wahlweise markiert ist, um einen dreikomponentigen Festphasen-Komplex zu bilden,
(s') wahlweise Zugeben zu dem dreikomponentigen Festphasen-Komplex einer Lösung von (iv) einer Markierungsverbindung, um einen vierkomponentigen Festphasen-Komplex zu bilden,
(t') Trennen des in Schritt (r') bzw. (s') erhaltenen drei- bzw. vierkomponentigen Festphasen-Komplexes von der Lösung,
(u') Waschen des abgetrennten drei- oder vierkomponentigen Festphasen-Komplexes, um nicht-Komplex-gebundene Verbindungen zu entfernen,
(v') Durchführen einer Detektion/Messung des gewaschenen markierten drei- oder vierkomponentigen Komplexes,

(y') Bestimmen des Gehalts an dem Antikörper unter Anwendung des folgenden Assays:

(ya') I3ereitstellen eines Gemischs aus einer Flüssigphase und einem vierkomponentigen Festphasen-Komplex, zusammengesetzt aus (i) dem Antikörper aus der Probe, (ii) einem Reaktant-Antikörper, der gegen den Proben-Antikörper gerichtet ist, wobei der Reaktant-Antikörper an einen festen Partikel gebunden ist, (iii) einem Liganden in der Form eines Antigens, eines Antikörpers oder eines Haptens, welcher/s markiert oder gebunden ist an (iv) eine Markierungsverbindung, um einen vielkomponentigen Festphasen-Komplex zu bilden,
(yb') Trennen des vielkomponentigen Festphasen-Komplexes von der Flüssigphase,
(yc') Waschen des abgetrennten vielkomponentigen Festphasen-Komplexes, um nicht-Komplex-gebundene Verbindungen zu entfernen,
(yd') Durchführen einer Detektion/Messung des gewaschenen markierten vielkomponentigen Komplexes,

(z') Vergleichen der Messungen, die in Schritt (x') und in Schritt (y') erhalten wurden, und Verwenden des Vergleichs zur Auswertung und/oder Vorhersage der Wirkung der spezifischen Allergie-Vakzinationsbehandlung.

**5.** Methode nach Anspruch 4,
wobei in Schritt (r') und in Schritt (ya') der Ligand an Biotin oder ein funktionelles Derivat davon gebunden ist, und wobei Schritt (s') obligatorisch ist und wobei in Schritt (s') und in Schritt (ya') die Markierungsverbindung eine chemilumineszierende Verbindung ist, die an Avidin, Streptavidin oder ein funktionelles Derivat davon kovalent gebunden ist, und
wobei in Schritt (v') und in Schritt (yd') die Detektion/Messung durch Initiieren einer chemilumineszierenden Reaktion in dem gewaschenen vierkomponentigen Festphasen-Komplex und Detektieren/Messen der resultierenden Chemilumineszenz, sofern vorhanden, durchgeführt wird.

**6.** Methode nach Anspruch 5,
wobei in Schritt (o') und in Schritt (ya') der feste Partikel ein fester paramagnetischer Partikel ist, und
wobei in Schritt (x'), in Schritt (t') und in Schritt (yb') die Trennung des Festphasen-Komplexes von der Flüssigphase magnetisch vorgenommen wird.

**7.** Methode nach Anspruch 1, 2, 3, 4, 5 oder 6, wobei Schritt (ia') bzw. (ya') durch Mischen der Komponenten (i) und (ii), dann Zugeben der Komponente (iii), und schließlich Zugeben der Komponente (iv), sofern diese zugegeben wird, vorgenommen wird.

**8.** Methode nach Anspruch 1, 2, 3, 4, 5 oder 6, wobei Schritt (ia') bzw. (ya') durch Mischen der Komponenten (i), (ii) und (iii), und dann Zugeben der Komponente (iv), sofern diese zugegeben wird, vorgenommen wird.

**9.** Methode nach Anspruch 1, 2, 3, 4, 5 oder 6, wobei der Vergleich von Schritt (j') und (z') durch Berechnen des Verhältnisses der Messungen, die in den beiden Schritten erhalten wurden, vorgenommen wird.

**10.** Methode nach einem der Ansprüche 1, 2, 3, 4, 5 oder 6, wobei der Vergleich von Schritts (j') und (z') zu einer Reihe von Zeitpunkten am Beginn und während der Behandlungsperiode durchgeführt wird, und wobei jegliche zeitliche Veränderung, die möglicherweise beobachtet wird, als eine Grundlage zum Auswerten und/oder Vorhersagen der Wirkung der Behandlung verwendet wird.

**11.** Methode zum Auswerten des immunologischen Status eines allergischen Individuums, umfassend die folgenden Schritte:

A) Bestimmen des Gehalts an einem Antikörper in einer Flüssigkeitsprobe von dem Individuum unter Anwendung des folgenden Assayprotokolls (Assay A):

(Aa) Mischen (i) des Antikörpers aus der Probe, (ii) eines Antikörpers, der gegen die Fc-Region des Proben-Antikörpers gerichtet ist, wobei der Reaktant-Antikörper an einen festen Träger gebunden ist, und (iii) eines Liganden in der Form eines Antigens, eines Antikörpers oder eines Haptens, wobei der Ligand auf die Fab-Region des Proben-Antikörpers gerichtet ist, um ein Gemisch aus einem dreikomponentigen Festphasen-Komplex und einer Festphase zu bilden,
(Ab) Kontaktieren des dreikomponentigen Komplexes mit (iv) einer Markierungsverbindung, um ein Gemisch aus einem vierkomponentigen Komplex und einer Festphase zu bilden,
(Ac) Waschen der vierkomponentigen Festphase, um nicht-Komplex-gebundene Verbindungen zu entfernen,
(Ad) Durchführen einer Detektion/Messung des gewaschenen markierten vierkomponentigen Komplexes, um eine Messung A zu erhalten;

(B) Bestimmen des Gehalts an dem Antikörper in der Probe unter Anwendung des folgenden Assayprotokolls (Assay B):

(Ba) Mischen (i) des Antikörpers aus der Probe, und (ii) eines Reaktant-Antikörpers, der gegen die Fc-Region des Proben-Antikörpers gerichtet ist, wobei der Reaktant-Antikörper an einen festen Träger gebunden ist, um ein Gemisch aus einem zweikomponentigen Festphasen-Komplex und einer Flüssigphase zu bilden,
(Bb) Waschen des zweikomponentigen Festphasen-Komplexes, um nicht-Komplex-gebundene Verbindungen zu entfernen,
(Bc) Kontaktieren des gewaschenen zweikomponentigen Festphasen-Komplexes mit (iii) einem Liganden in der Form eines Antigens, eines Antikörpers oder eines Haptens, wobei der Ligand an die Fab-Region des Proben-Antikörpers gebunden ist, und (iv) einer Markierungsverbindung, um ein Gemisch aus einem vierkomponentigen Festphasen-Komplex und einer Flüssigphase zu bilden,
(Bd) Waschen des vierkomponentigen Festphasen-Komplexes, um nicht-Komplex-gebundene Verbindungen zu entfernen,
(Be) Durchführen einer Detektion/Messung des gewaschenen markierten vierkomponentigen Komplexes, um eine Messung B zu erhalten; und

(E) Zueinander in Beziehung setzen der Messungen A und B, um die Beeinträchtigung auszudrücken, und Verwenden der Beeinträchtigung als einen Parameter zum Auswerten des immunologischen Status des Individuums.

**12.** Methode nach Anspruch 11, wobei Schritt Ab ausgeführt wird durch Zugeben der (iv) Markierungsverbindung zu dem Gemisch aus einem dreikomponentigen Festphasen-Komplex und einer Flüssigphase in Schritt Aa.

**13.** Methode nach Anspruch 11, wobei Schritt Ab ausgeführt wird durch Waschen des in Schritt Aa erhaltenen dreikomponentigen Komplexes, um nicht-Komplex-gebundene Verbindungen zu entfernen, und durch anschließendes Zugeben der (iv) Markierungsverbindung zu dem gewaschenen dreikomponentigen Komplex.

**14.** Methode nach einem der Ansprüche 11-13, wobei Schritt Bc ausgeführt wird durch gleichzeitiges Inkubieren des (iii) Liganden und der (iv) Markierungsverbindung mit dem zweikomponentigen Komplex.

**15.** Methode nach einem der Ansprüche 11-13, wobei anfänglich der (iii) Ligand dem zweikomponentigen Komplex zugegeben wird, um einen dreikomponentigen Komplex zu bilden, welcher dann gewaschen wird, um nicht-Komplexgebundenen (iii) Ligand zu entfernen, und dann die (iv) Markierungsverbindung zugegeben wird, um einen vierkomponentigen Komplex zu bilden.

**16.** Methode nach einem der Ansprüche 11-15, wobei die Markierungsverbindung eine lumineszierende Markierung, eine chemilumineszierende Markierung, eine Enzymmarkierung, eine radioaktive Markierung, eine fluoreszierende Markierung oder eine absorbierende Markierung ist.

**17.** Methode nach einem der Ansprüche 11-16, wobei der (iii) Ligand biotinyliert ist.

**18.** Methode nach Anspruch 17, wobei die (iv) Markierungsverbindung eine chemilumineszierende Verbindung ist, die an Avidin, Streptavidin oder ein funktionelles Derivat davon kovalent gebunden ist.

**19.** Methode zum Auswerten des immunologischen Status eines allergischen Individuums, umfassend die folgenden Schritte:

C) Bestimmen des Gehalts an einem Antikörper in einer Flüssigkeitsprobe von dem Individuum unter Anwendung des folgenden Assayprotokolls (Assay C):
(Ca) Mischen (i) des Antikörpers aus der Probe, (ii) eines Antikörpers, der gegen die Fc-Region des Proben-Antikörpers gerichtet ist, wobei der Reaktant-Antikörper an einen festen Träger gebunden ist, und (iii) eines markierten Liganden in der Form eines Antigens, eines Antikörpers oder eines Haptens, wobei der Ligand auf die Fab-Region des Proben-Antikörpers gerichtet ist, um ein Gemisch aus einem dreikomponentigen Festphasen-Komplex und einer Festphase zu bilden,
(Cb) Waschen des dreikomponentigen Festphasen-Komplexes, um nicht-Komplex-gebundene Verbindungen zu entfernen,
(Cc) Durchführen einer Detektion/Messung des gewaschenen markierten dreikomponentigen Komplexes, um eine Messung C zu erhalten;
(D) Bestimmen des Gehalts an dem Antikörper unter Anwendung des folgenden Assayprotokolls (Assay D):
(Da) Mischen (i) des Antikörpers aus der Probe, und (ii) eines Reaktant-Antikörpers, der gegen die Fc-Region des Proben-Antikörpers gerichtet ist, wobei der Reaktant-Antikörper an einen festen Träger gebunden ist, um ein Gemisch aus einem zweikomponentigen Festphasen-Komplex und einer Flüssigphase zu bilden,
(Db) Waschen des zweikomponentigen Festphasen-Komplexes, um nicht-Komplex-gebundene Verbindungen zu entfernen,
(Dc) Kontaktieren des gewaschenen zweikomponentigen Festphasen-Komplexes mit (iii) einem markierten Liganden in der Form eines Antigens, eines Antikörpers oder eines Haptens, wobei der Ligand an die Fab-Region des Proben-Antikörpers gebunden ist, um ein Gemisch aus einem dreikomponentigen Festphasen-Komplex und einer Festphase zu bilden,
(Dd) Waschen des dreikomponentigen Festphasen-Komplexes, um nicht-Komplex-gebundene Verbindungen zu entfernen,
(De) Durchführen einer Detektion/Messung des gewaschenen markierten dreikomponentigen Komplexes, um eine Messung D zu erhalten; und
(E) Zueinander in Beziehung setzen der Messungen C und D, um die Beeinträchtigung auszudrücken, und Verwenden der Beeinträchtigung als einen Parameter zum Auswerten des immunologischen Status des Individuums.

**20.** Methode nach Anspruch 19, wobei der markierte Ligand durch ein radioaktives Atom markiert ist.

**21.** Methode nach einem der Ansprüche 11-20, wobei das auszuwertende Individuum einer Allergiebehandlung, Allergie-Vakzinationsbehandlung oder spezifischen Allergie-Vakzinations-(SAV)-Behandlung unterzogen wird.

## Revendications

**1.** Procédé d'évaluation et/ou de prédiction de l'effet d'un traitement de vaccination spécifique contre l'allergie comprenant les stades dans lesquels :

(h') on détermine la teneur en un anticorps d'un échantillon liquide en utilisant le dosage suivant :

(a') on se procure un mélange d'une phase liquide et d'un complexe à trois constituants en phase solide composé de (i) l'anticorps de l'échantillon (ii) un anticorps de réactif dirigé à l'encontre de l'anticorps de l'échantillon, l'anticorps de réactif étant fixé à une particule solide et (iii) un ligand sous la forme d'un antigène, d'un anticorps ou d'un haptène,
(b') on sépare le complexe à trois constituants en phase solide de la phase liquide,

(c') on lave le complexe séparé à trois constituants en phase solide pour éliminer des composés fixés non complexes,
(d') on ajoute au complexe à trois constituants en phase solide une solution de (iv) un composé marqueur pour former un complexe à quatre constituants,
(e') on sépare le complexe à quatre constituants en phase solide de la solution,
(f') on lave le complexe séparé à quatre constituants en phase solide pour éliminer un composé fixé non complexe (iv),
(g') on effectue une détection/mesure du complexe à quatre constituants lavé et marqué,

i') on détermine la teneur en ledit anticorps en utilisant le dosage suivant :

(ia') on se procure un mélange d'une phase liquide et d'un complexe à quatre constituants en phase solide composé de (i), l'anticorps de l'échantillon (ii), un anticorps de réactif dirigé contre l'anticorps de l'échantillon, l'anticorps de réactif étant fixé à une particule solide (iii) un ligand sous la forme d'un antigène, d'un anticorps ou d'un haptène, et (iv) un composé marqueur pour former un complexe à quatre constituants en phase solide,
(ib') on sépare le complexe à quatre constituants en phase solide de la phase liquide,
(ic') on lave le complexe séparé à quatre constituants en phase solide pour éliminer des composés fixés non complexes,
(id') on effectue une détection/mesure du complexe à quatre constituants lavé et marqué,

(j') on compare les mesures obtenues au stade (h') et au stade (i') et on utilise la comparaison pour évaluer et/ou prédire l'effet d'un traitement de vaccination spécifique contre l'allergie.

**2.** Procédé suivant la revendication 1,
dans lequel dans le stade (a') et dans le stade (ia') le ligand est fixé à de la biotine ou à l'un de ses dérivés fonctionnels, et
dans lequel dans le stade (d') et dans le stade (ia') le composé marqueur est un composé chémioluminescent lié par covalence à de l'avidine, de la streptavidine ou à l'un de leurs dérivés fonctionnels, et dans lequel dans le stade (g') et dans le stade (id') on effectue la détection/mesure en amorçant une réaction chémioluminescente dans le complexe lavé à quatre constituants en phase solide et en détectant/mesurant la chémioluminescence obtenue s'il y en a une.

**3.** Procédé suivant la revendication 2,
dans lequel dans le stade (a') et dans le stade (ia') la particule solide est une particule solide paramagnétique et dans lequel dans le stade (b') et dans le stade (ib'), on effectue la séparation du complexe en phase solide de la phase liquide par voie magnétique.

**4.** Procédé d'évaluation et/ou de prédiction de l'effet d'un traitement de vaccination spécifique contre l'allergie, comprenant les stades dans lesquels :

(x') on détermine la teneur en anticorps d'un échantillon liquide en utilisant le procédé suivant :

(o') on se procure le mélange d'une phase liquide et d'un complexe à deux constituants en phase solide, composé de (i) l'anticorps de l'échantillon et (ii) un anticorps de réactif dirigé contre l'anticorps de l'échantillon, l'anticorps de réactif étant fixé à une particule solide,
(p') on sépare le complexe à deux constituants en phase solide de la phase liquide,
(q') on lave le complexe séparé à deux constituants en phase solide pour éliminer des composés fixés non complexes,
(r') on ajoute au complexe lavé à deux constituants en phase solide une solution (iii), un ligand sous la forme d'un antigène ou d'un anticorps ou d'un haptène qui est éventuellement marqué pour former un complexe à trois constituants en phase solide,
(s') on ajoute éventuellement au complexe à trois constituants en phase solide une solution de (iv) un composé marqueur pour former un complexe à quatre constituants en phase solide,
(t') on sépare le complexe à trois constituants en quatre constituants en phase solide du stade (r') ou (s') respectivement de la solution,
(u') on lave le complexe séparé à trois constituants ou à quatre constituants en phase solide pour éliminer des composés fixés non complexes,

(v') on effectue une détection/mesure du complexe à trois ou à quatre constituants lavé et marqué,
(y') on détermine la teneur en ledit anticorps en utilisant le dosage suivant :
(ya') on se procure un mélange d'une phase liquide et d'un complexe à trois constituants en phase solide composé de (i) l'anticorps de l'échantillon (ii) un anticorps de réactif dirigé contre l'anticorps de l'échantillon l'anticorps de réactif étant fixé à une particule solide,
(iii) un ligand sous la forme d'un antigène, d'un anticorps ou d'un haptène qui est marqué ou fixé à (iv) un composé marqueur pour former un complexe à plusieurs constituants en phase solide,
(yb') on sépare le complexe à plusieurs constituants en phase solide de la phase liquide,
(yc') on lave le complexe séparé à plusieurs constituants en phase solide pour éliminer des composés fixés non complexe,
(yd') on effectue une détection/mesure du complexe à plusieurs constituants lavé et marqué,

(z') on compare les mesures obtenues au stade (x') et au stade (y') et on utilise la comparaison pour évaluer et/ou prédire l'effet du traitement de vaccination spécifique contre l'allergie.

**5.** Procédé suivant la revendication 4,
dans lequel dans le stade (r') et dans le stade (ya') le ligand est fixé à la biotine ou à l'un de ses dérivés fonctionnels, et dans lequel le stade (s') est obligatoire et dans lequel dans le stade (s') et dans le stade (ya') le composé marqueur est un composé chémioluminescent lié par covalence à de l'avidine, à de la streptavidine ou à l'un de leurs dérivés fonctionnels, et
dans le stade (v') et dans le stade (yd') on effectue la détection/mesure en amorçant une réaction de chémioluminescence dans le complexe lavé à quatre constituants en phase solide et en détectant/mesurant la chémioluminescence obtenue s'il y en a une.

**6.** Procédé suivant la revendication 5,
dans lequel dans le stade (o') et dans le stade (ya') la particule solide est une particule solide paramagnétique, et dans lequel dans le stade (x') et dans le stade (t') et dans le stade (yb') on effectue la séparation du complexe en phase solide de la phase liquide en voie magnétique.

**7.** Procédé suivant la revendication 1, 2, 3, 4, 5 ou 6, dans lequel on effectue le stade (ia') et le stade (ya') respectivement en mélangeant des constituants (i) et (ii), puis en ajoutant le constituant (iii) et en ajoutant finalement le constituant (iv) s'il est ajouté.

**8.** Procédé suivant la revendication 1, 2, 3, 4, 5 ou 6, dans lequel on effectue le stade (ia') ou (ya') respectivement en mélangeant des constituants (i), (ii) et (iii) puis en ajoutant le constituant (iv), s'il est ajouté.

**9.** Procédé suivant la revendication 1, 2, 3, 4, 5 ou 6, dans lequel on effectue la comparaison du stade (j') ou (z') respectivement en calculant le rapport des mesures obtenues dans les deux stades.

**10.** Procédé suivant l'une quelconque des revendications 1, 2, 3, 4, 5 ou 6, dans lequel on effectue la comparaison du stade (j') ou (z') respectivement en un certain nombre de points dans le temps au début et pendant la durée du traitement et en ce que l'on utilise toute variation dans le temps qui peut être observée comme base pour évaluer et/ou prédire l'effet du traitement.

**11.** Procédé d'évaluation du statut immunologique d'un sujet allergique comprenant les stades dans lesquels

(A) on détermine la teneur en un anticorps d'un échantillon liquide provenant du sujet en utilisant le protocole de dosage suivant (dosage A) :
(Aa) on mélange (i) l'anticorps de l'échantillon (ii) un anticorps dirigé contre la région Fc de l'anticorps de l'échantillon, l'anticorps du réactif étant fixé à un support solide et (iii) un ligand sous la forme d'un antigène, ou d'un anticorps ou d'un haptène le ligand étant dirigé sur la région Fab de l'anticorps de l'échantillon pour former un mélange d'un complexe à trois constituants en phase solide et d'une phase liquide,
(Ab) on met le complexe à trois constituants en contact avec (iv) un composé marqueur pour former un mélange d'un complexe à quatre constituants et d'une phase liquide,
(Ac) on lave la phase solide à quatre constituants pour éliminer des composés fixés non complexes,
(Ad) on effectue une détection/mesure du complexe à quatre constituants lavé et marqué pour obtenir une mesure A ;
B) on détermine la teneur en ledit anticorps de l'échantillon en utilisant le protocole de dosage suivant (dosage B) :

(Ba) on mélange (i) l'anticorps de l'échantillon et (ii) un anticorps de réactif dirigé contre la région Fc de l'anticorps de l'échantillon, l'anticorps du réactif étant fixé à un support solide pour former un mélange d'un complexe à deux constituants en phase solide et d'une phase liquide,
(Bb) on lave le complexe à deux constituants en phase solide pour éliminer des composés fixés non complexes,
(Bc) on met le complexe lavé à deux constituants en phase solide en contact avec (iii) un ligand sous la forme d'un antigène, d'un anticorps ou d'un haptène, le ligand étant fixé à la région Fab de l'anticorps de l'échantillon et (iv) un composé marqueur pour former un mélange d'un complexe à quatre constituants en phase solide et d'une phase liquide,
(Bd) on lave le complexe à quatre constituants en phase solide pour éliminer des composés fixés non complexes,
(Be) on effectue une détection/mesure du complexe à quatre constituants lavés et marqués pour obtenir une mesure B ; et
(E) on met en relation les mesures A et B pour exprimer l'interférence et on utilise l'interférence en tant que paramètre du statut immunologique du sujet.

**12.** Procédé suivant la revendication 11, dans lequel on effectue le stade Ab en ajoutant le (iv) composé marqueur audit mélange d'un complexe à trois constituants en phase solide et d'une phase liquide dans le stade Aa.

**13.** Procédé suivant la revendication 11, dans lequel on effectue le stade Ab en lavant le complexe à trois constituants obtenu dans le stade Aa pour éliminer des composés fixés non complexes et en ajoutant ensuite ledit (iv) composé marqueur au complexe lavé à trois constituants.

**14.** Procédé suivant l'une quelconque des revendications 11 à 13, dans lequel on effectue le stade Bc par incubation simultanée dudit (iii) ligand et dudit (iv) composé marqueur avec le complexe à deux constituants.

**15.** Procédé suivant l'une quelconque des revendications 11 à 13, dans lequel on ajoute initialement ledit (iii) ligand au complexe à deux constituants pour former un complexe à trois constituants qu'on lave ensuite pour éliminer (iii) du ligand fixé non complexe puis on ajoute (iv) ledit composé marqueur pour former un complexe à quatre constituants.

**16.** Procédé suivant l'une quelconque des revendications 11 à 15, dans lequel le composé marqueur est un marqueur luminescent, un marqueur chémioluminescent, un marqueur enzymatique, un marqueur de radioactivité, un marqueur fluorescent ou un marqueur d'absorbance.

**17.** Procédé suivant l'une quelconque des revendications 11 à 16, dans lequel le (iii) ligand est biotinylé.

**18.** Procédé suivant la revendication 17, dans lequel le (iv) composé marqueur est un composé chémioluminescent lié par covalence à de l'avidine, à de la streptavidine ou à l'un de leurs dérivés fonctionnels.

**19.** Procédé d'évaluation du statut immunologique d'un sujet allergique comprenant les stades dans lesquels :

C) on détermine la teneur en anticorps d'un échantillon liquide provenant du sujet en utilisant le protocole de dosage suivant (dosage C) :
(Ca) on mélange (i) l'anticorps de l'échantillon (ii) un anticorps dirigé contre la région Fc de l'anticorps de l'échantillon, l'anticorps du réactif étant fixé à un support solide et (iii), un ligand marqué sous la forme d'un antigène, d'un anticorps ou d'un haptène, le ligand étant dirigé sur la région Fab de l'anticorps de l'échantillon pour former un mélange d'un complexe à trois constituants en phase solide et d'une phase liquide,
(Cb) on lave la phase solide à trois constituants pour éliminer des composés fixés non complexes,
(Cc) on effectue une détection/mesure du complexe à trois constituants lavés et marqués pour obtenir une mesure C ;
D) on détermine la teneur dudit anticorps en utilisant le protocole de dosage suivant (dosage D) :
(Da) on mélange (i) l'anticorps de l'échantillon et (ii) un anticorps de réactif dirigé contre la région Fc de l'anticorps de l'échantillon, l'anticorps du réactif étant fixé à un support solide pour former un mélange d'un complexe à deux constituants en phase solide et d'une phase liquide,
(Db) on lave le complexe à deux constituants en phase solide pour éliminer des composés fixés et non complexes,
(Dc) on met le complexe lavé à deux constituants en phase solide en contact avec (iii) un ligand marqué sous la forme d'un antigène, d'un anticorps ou d'un haptène, le ligand étant fixé à la région Fab de l'anticorps de l'échantillon pour former un mélange d'un complexe à trois constituants en phase solide et d'une phase liquide,
(Dd) on lave le complexe à trois constituants en phase solide pour éliminer des composés fixés et non complexes,
(De) on effectue une détection/mesure du complexe à trois constituants lavé et marqué pour obtenir une mesure D ; et

(E) on met en relation les mesures C et D pour exprimer l'interférence et on utilise l'interférence comme paramètre d'évaluation du statut immunologique du sujet.

**20.** Procédé suivant la revendication 19, dans lequel le ligand marqué est marqué par un atome radioactif.

**21.** Procédé suivant l'une quelconque des revendications 11 à 20, dans lequel le sujet à évaluer est soumis à un traitement comme l'allergie, à un traitement de vaccination contre l'allergie ou à un traitement de vaccination spécifique contre l'allergie (SAV).

# Figure 1a

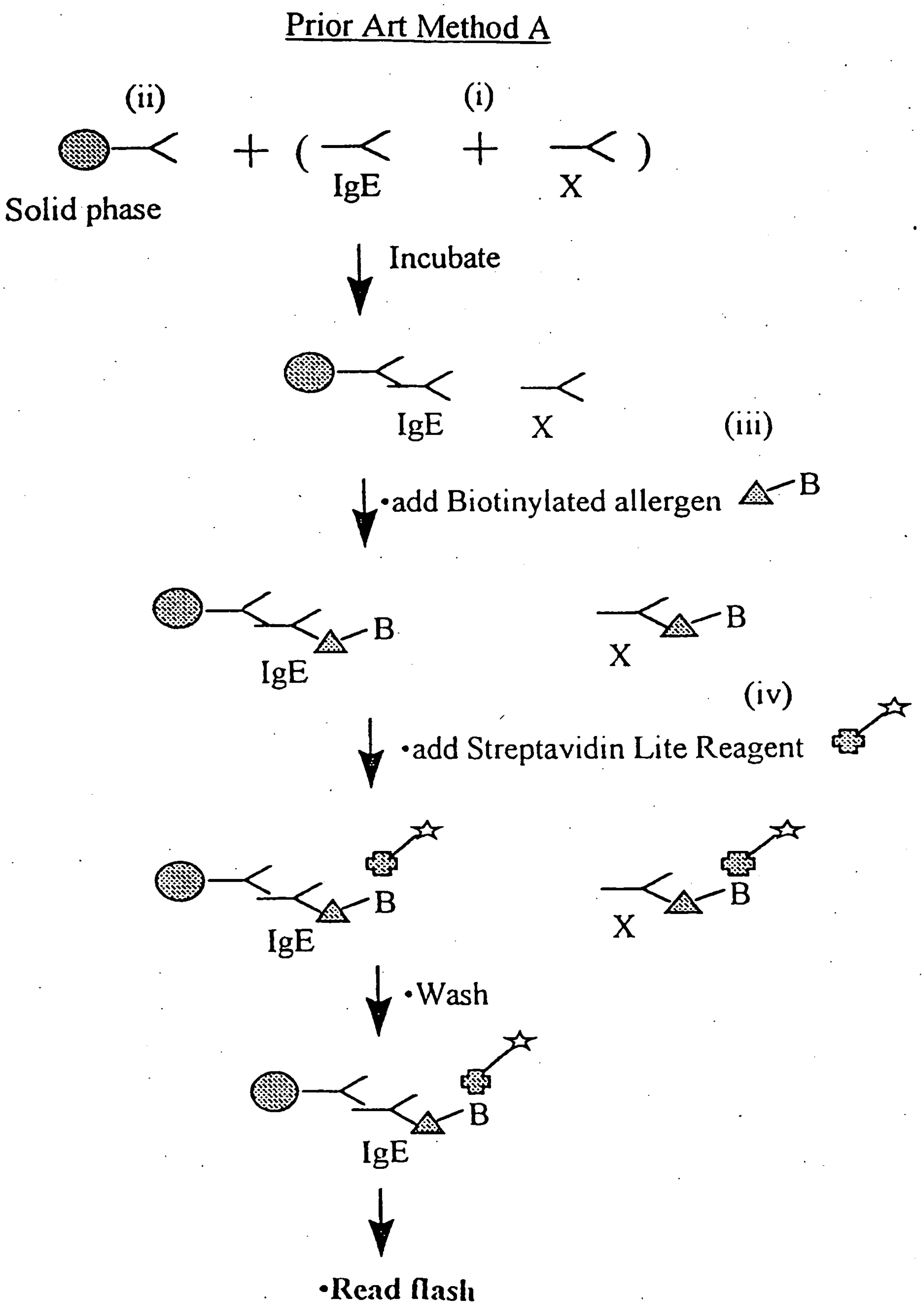
Prior Art Method A
(ii)
(i)
Solid phase
IgE
X
Incubate
IgE
X
(iii)
•add Biotinylated allergen
B
B
IgE
X
B
(iv)
•add Streptavidin Lite Reagent
B
IgE
X
B
•Wash
B
IgE
•Read flash

## Figure 1b

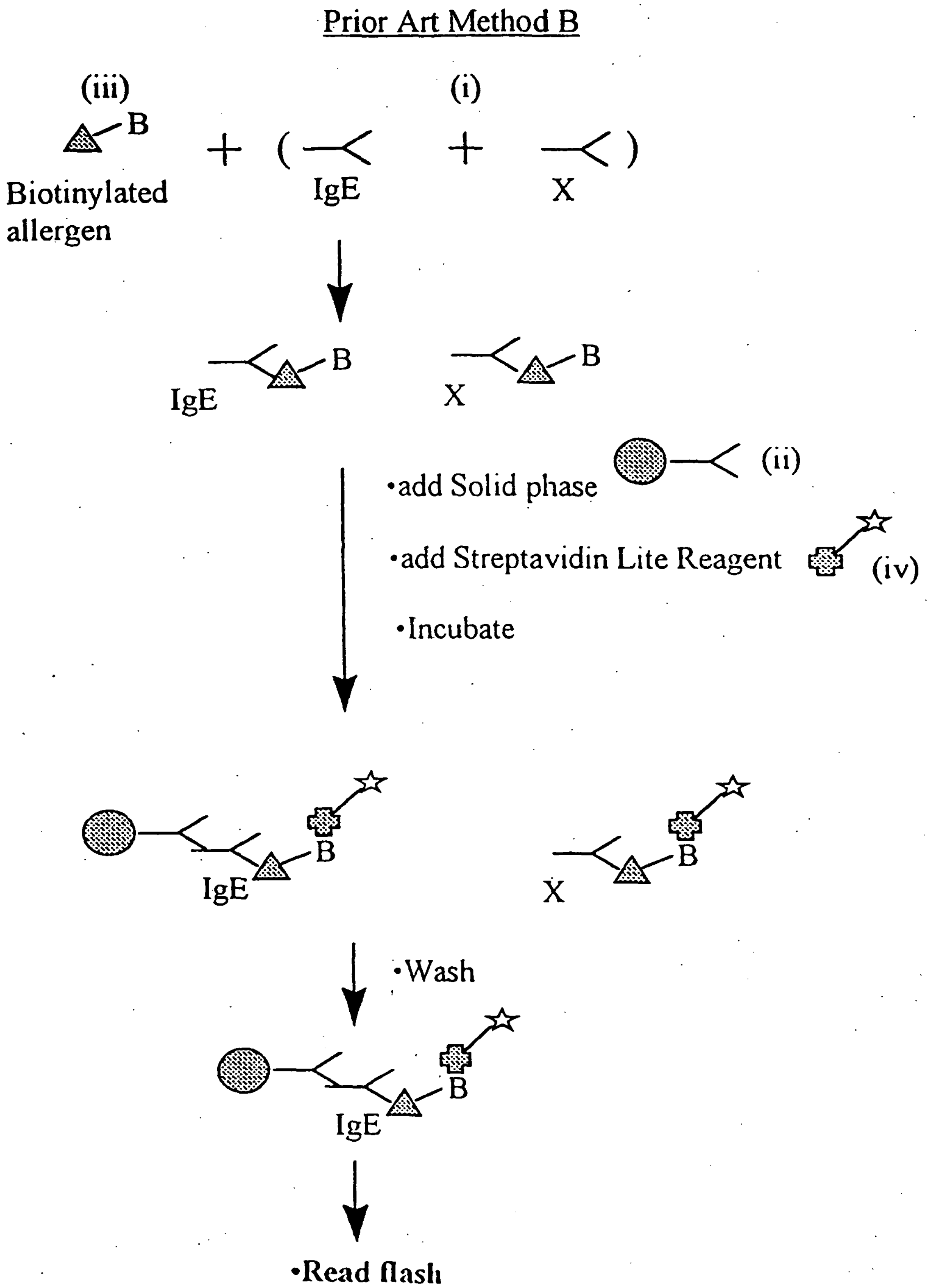
Prior Art Method B
(iii)
B
Biotinylated allergen
(i)
IgE
X
B
IgE
B
X
•add Solid phase
(ii)
•add Streptavidin Lite Reagent
(iv)
•Incubate
B
IgE
B
X
•Wash
B
IgE
•Read flash

## Figure 2a

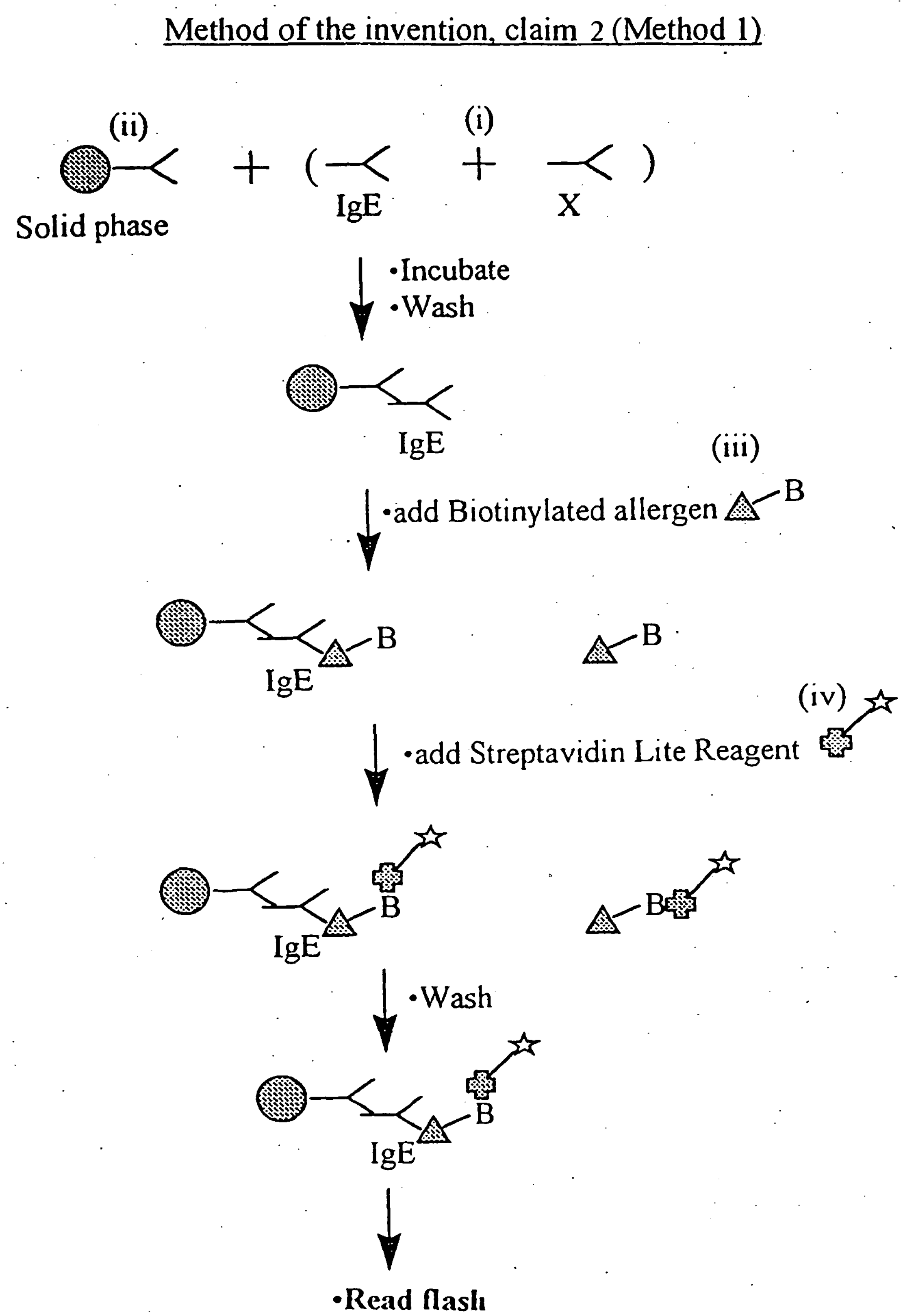
Method of the invention, claim 2 (Method 1)
(ii)
(i)
Solid phase
IgE
X
•Incubate
•Wash
IgE
(iii)
•add Biotinylated allergen
B
IgE
B
B
(iv)
•add Streptavidin Lite Reagent
B
IgE
B
•Wash
B
IgE
•Read flash

## Figure 2b

Method of the invention, claim 5

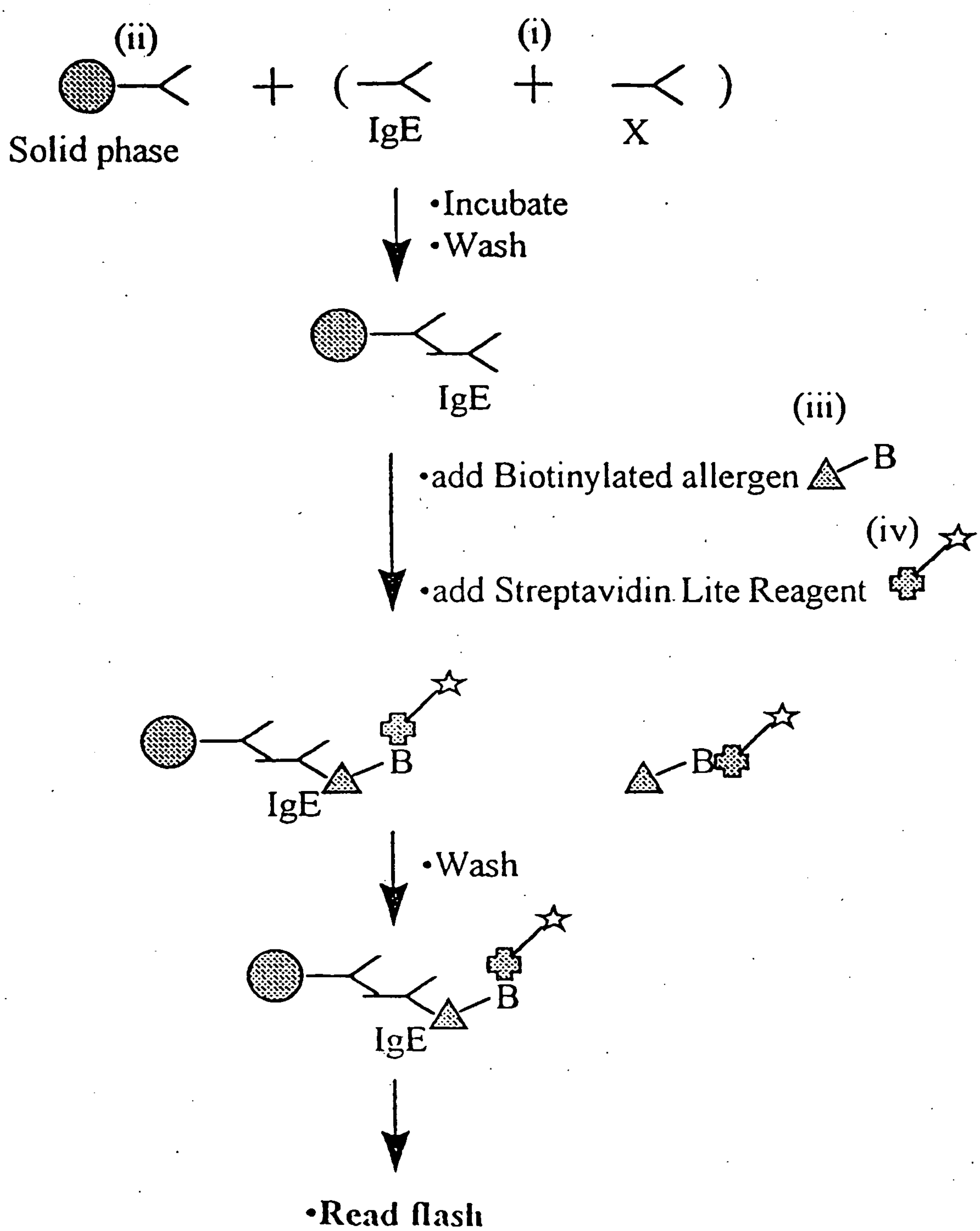

## Figure 2c

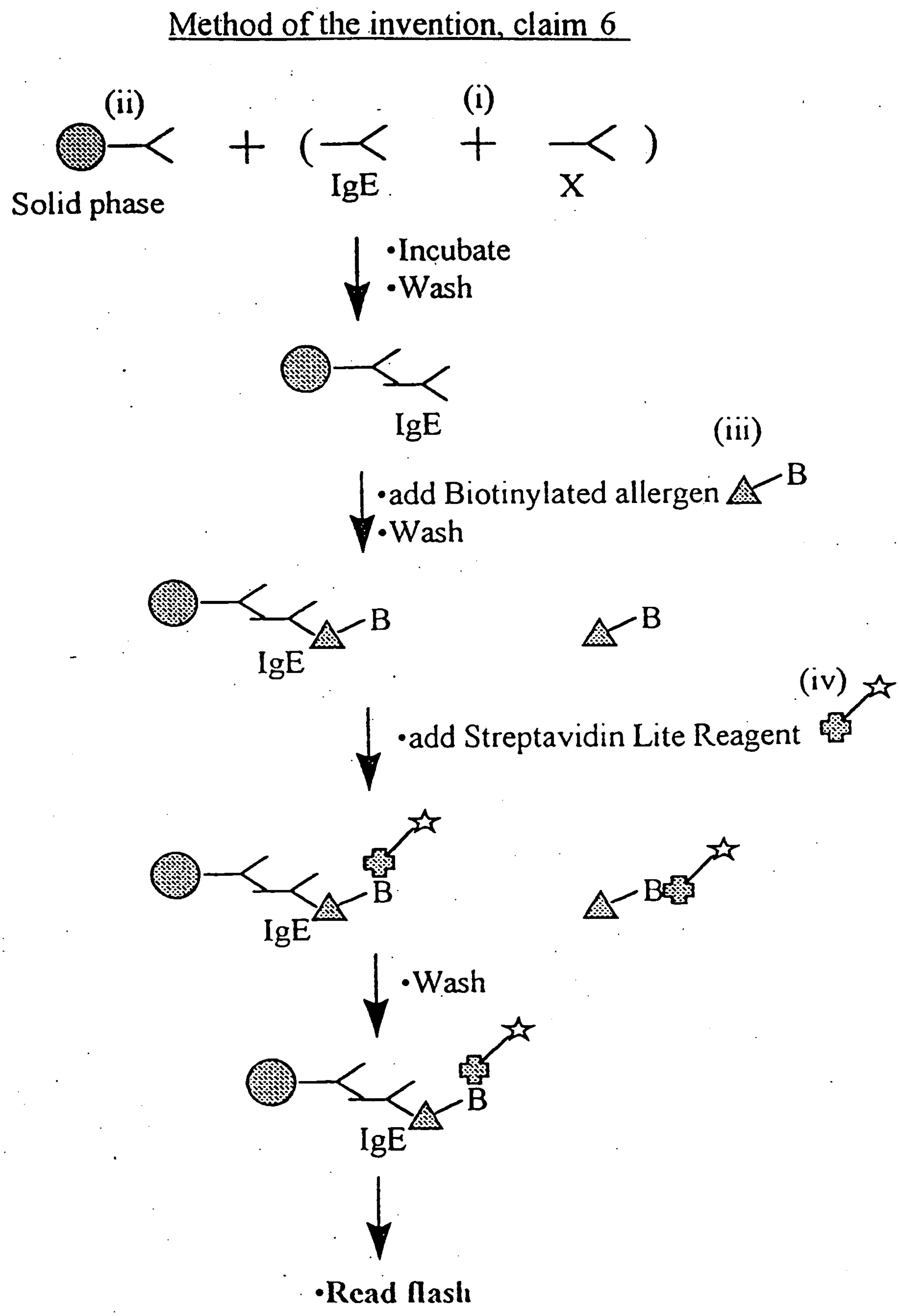
Method of the invention, claim 6
(ii)
(i)
Solid phase
IgE
X
•Incubate
•Wash
IgE
(iii)
•add Biotinylated allergen
B
•Wash
IgE
B
B
(iv)
•add Streptavidin Lite Reagent
IgE
B
B
•Wash
IgE
B
•Read flash

# Figure 3a

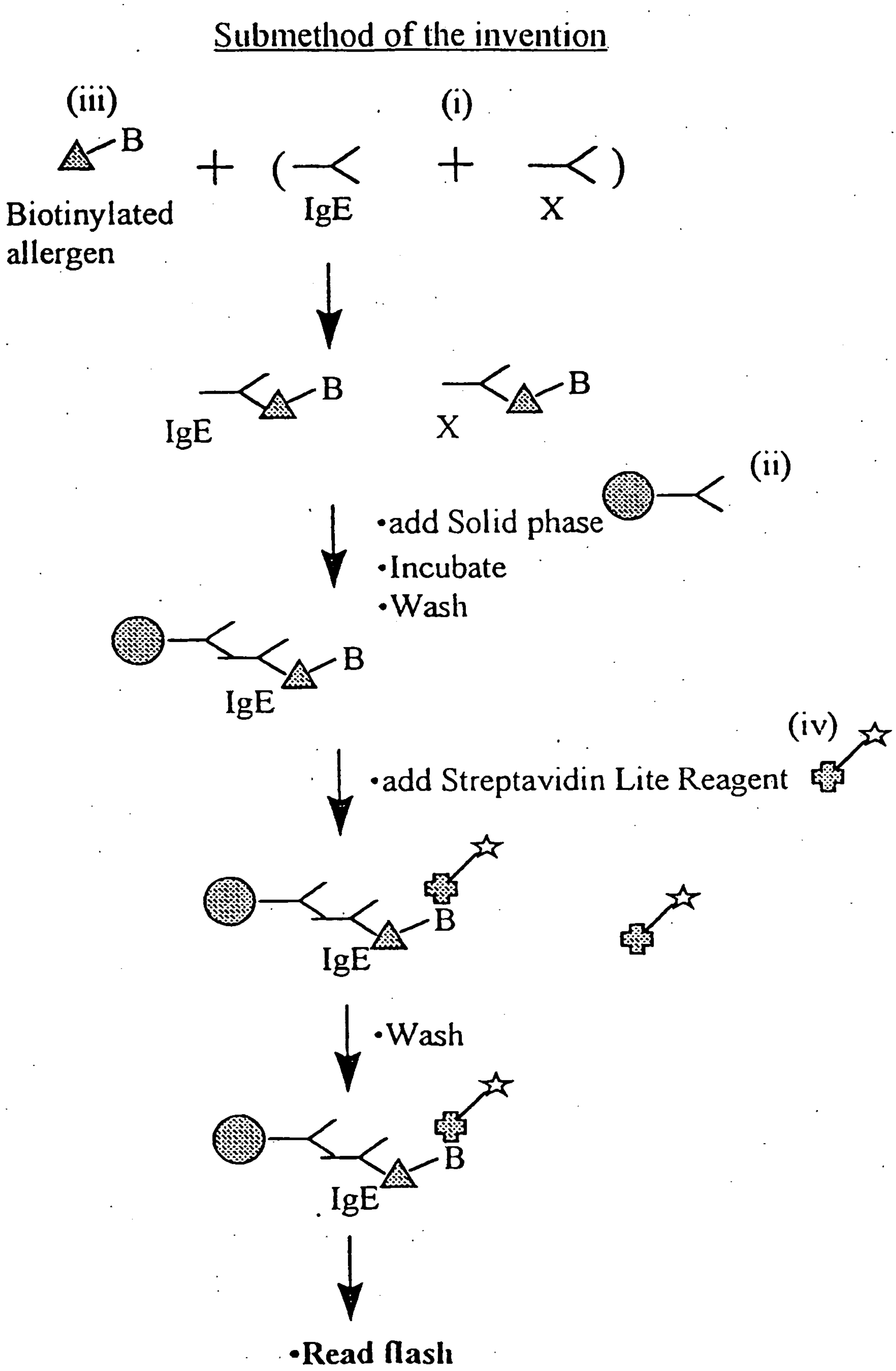
Submethod of the invention
(iii)
B
Biotinylated allergen
(i)
IgE
X
IgE
B
X
B
(ii)
•add Solid phase
•Incubate
•Wash
IgE
B
(iv)
•add Streptavidin Lite Reagent
IgE
B
•Wash
IgE
B
•Read flash

# Figure 3b

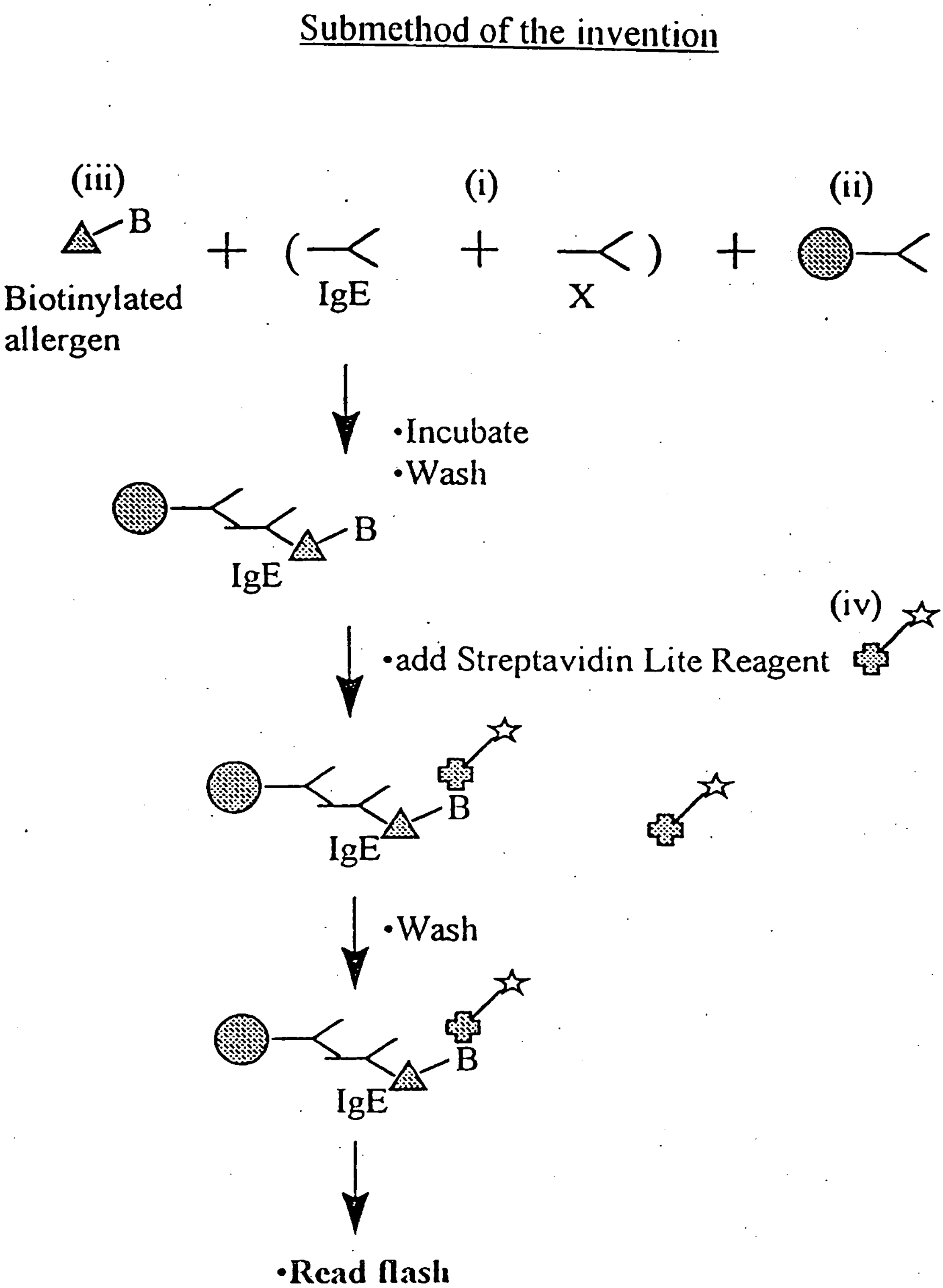

Submethod of the invention
(iii)
B
Biotinylated allergen
(i)
IgE
X
(ii)
•Incubate
•Wash
B
IgE
(iv)
•add Streptavidin Lite Reagent
B
IgE
•Wash
B
IgE
•Read flash

# Figure 3c

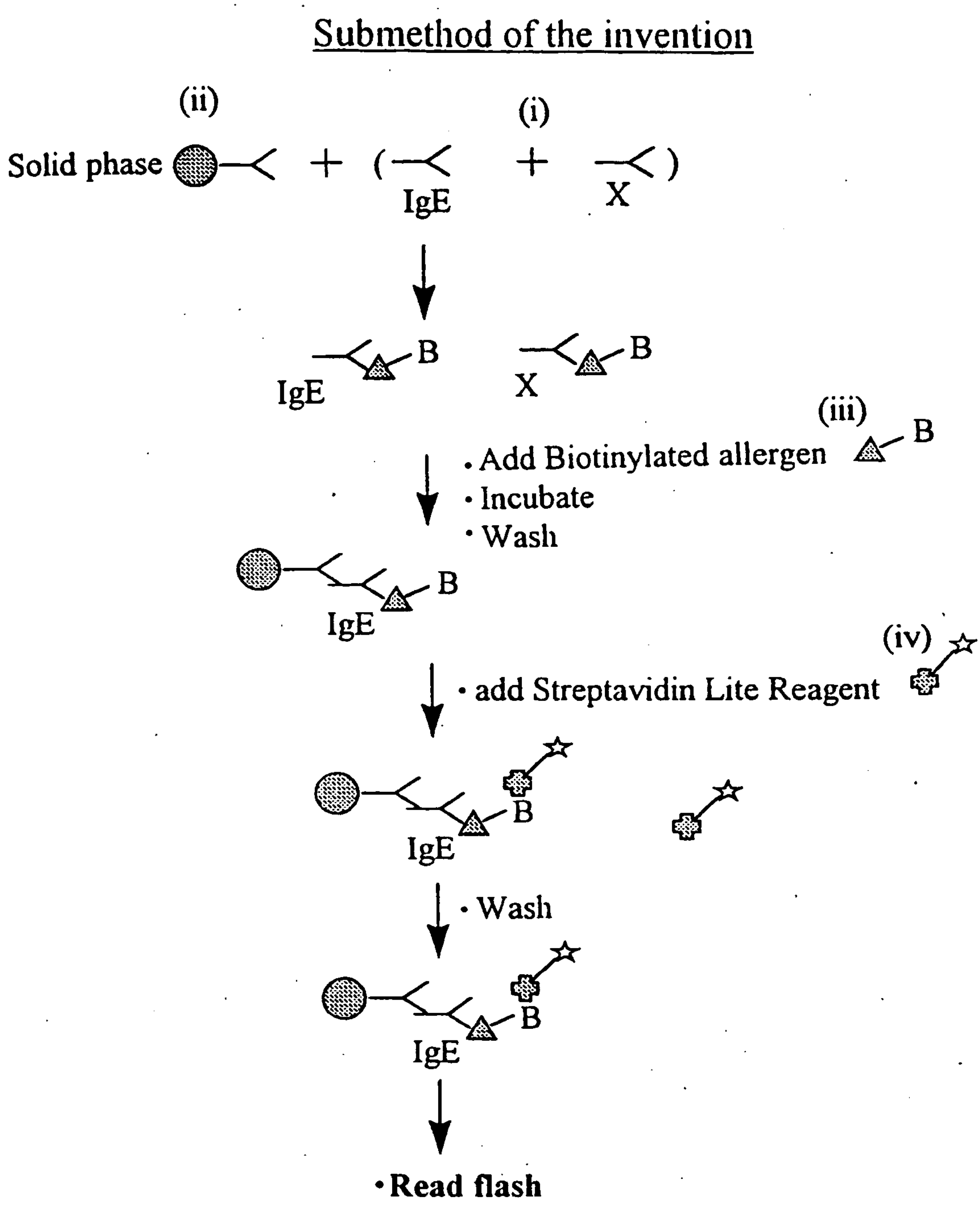
Submethod of the invention
(ii)
(i)
Solid phase
IgE
X
B
IgE
X
B
(iii)
B
• Add Biotinylated allergen
• Incubate
• Wash
B
IgE
(iv)
• add Streptavidin Lite Reagent
B
IgE
• Wash
B
IgE
• Read flash

Figure 4

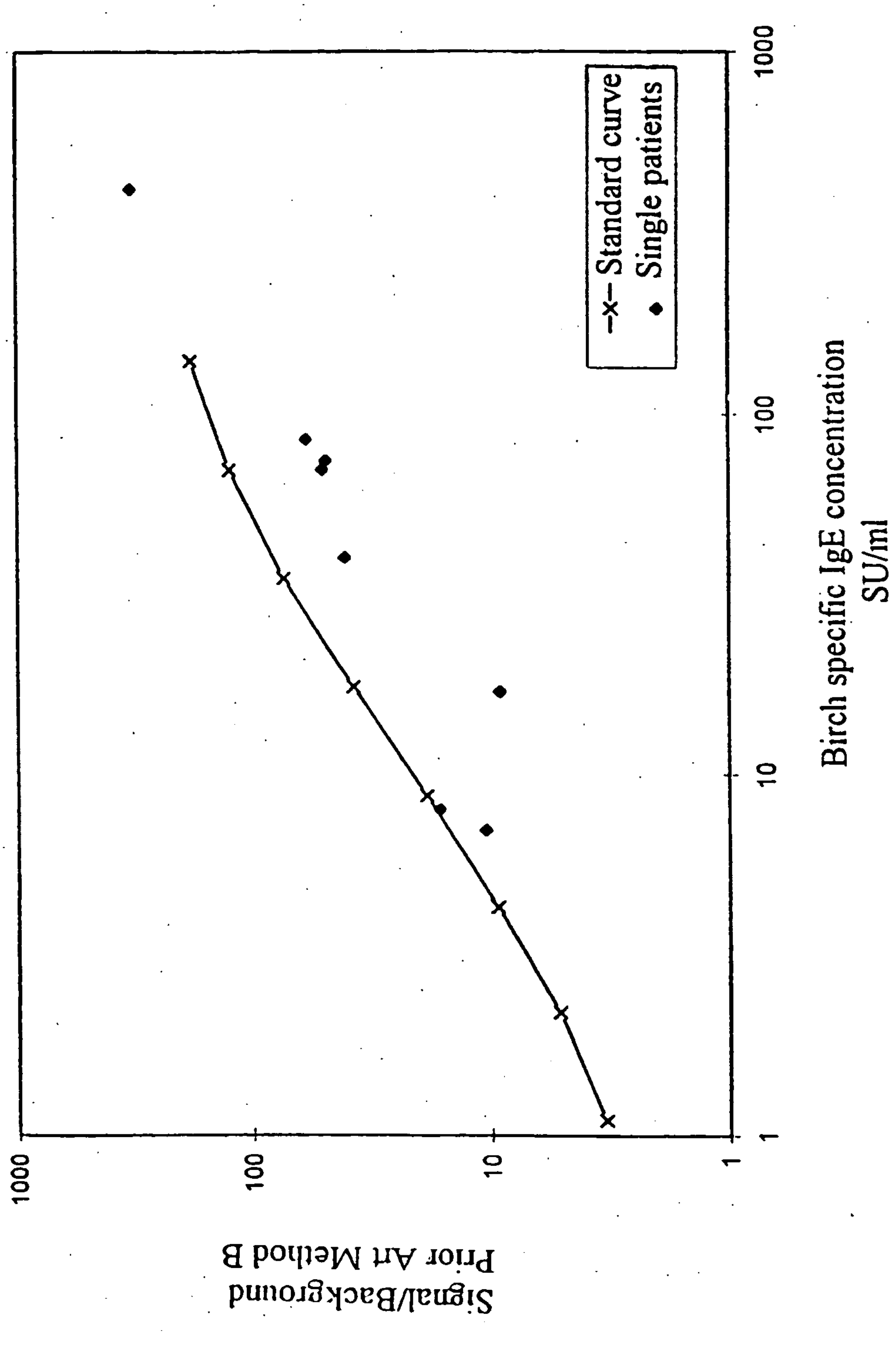
Signal/Background
Prior Art Method B
1000
100
10
1
1
10
100
1000
Birch specific IgE concentration
SU/ml
Standard curve
Single patients

Figure 5

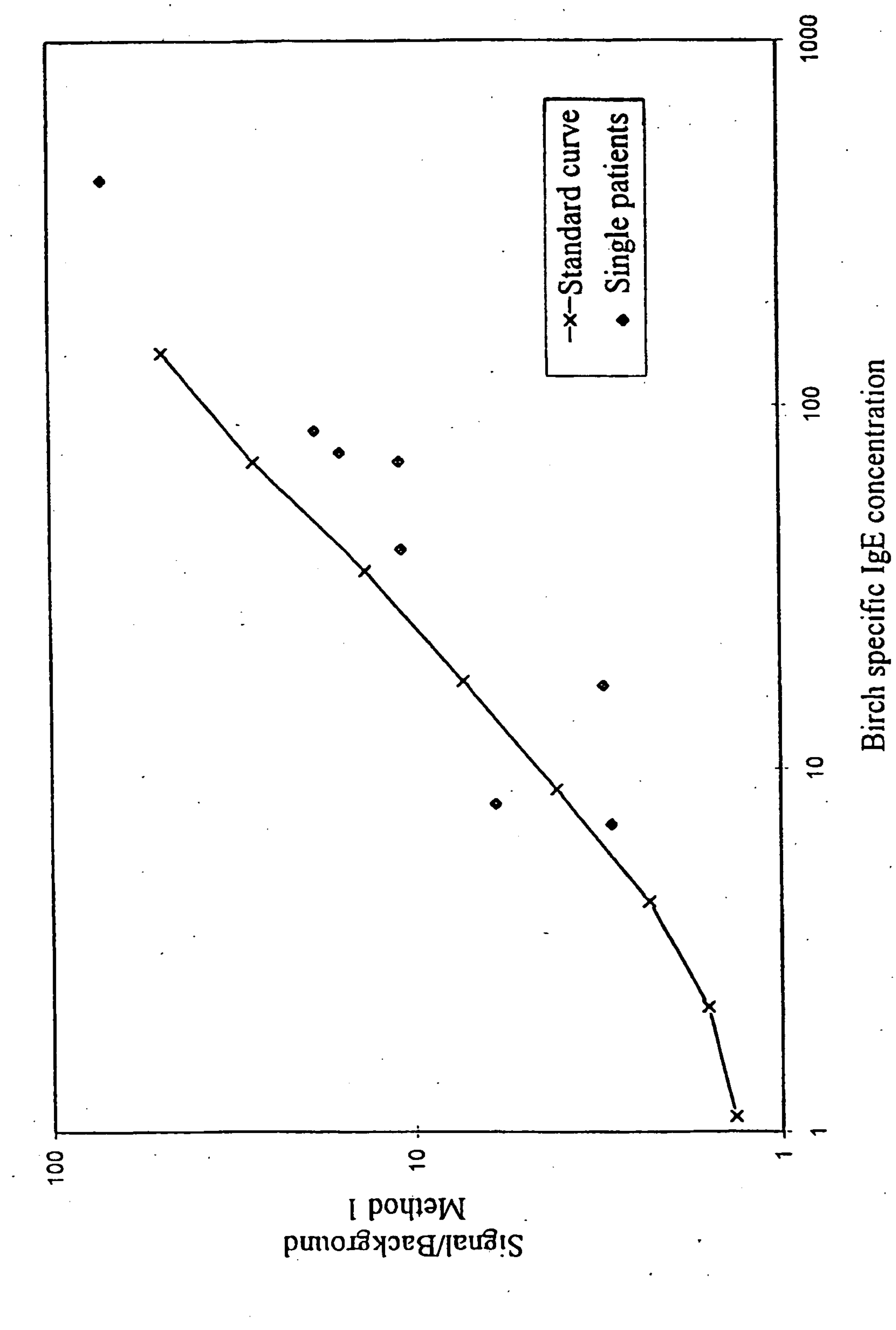

100
10
1
Signal/Background
Method 1
1
10
100
1000
Birch specific IgE concentration
SU/ml
Standard curve
Single patients

Figure 6

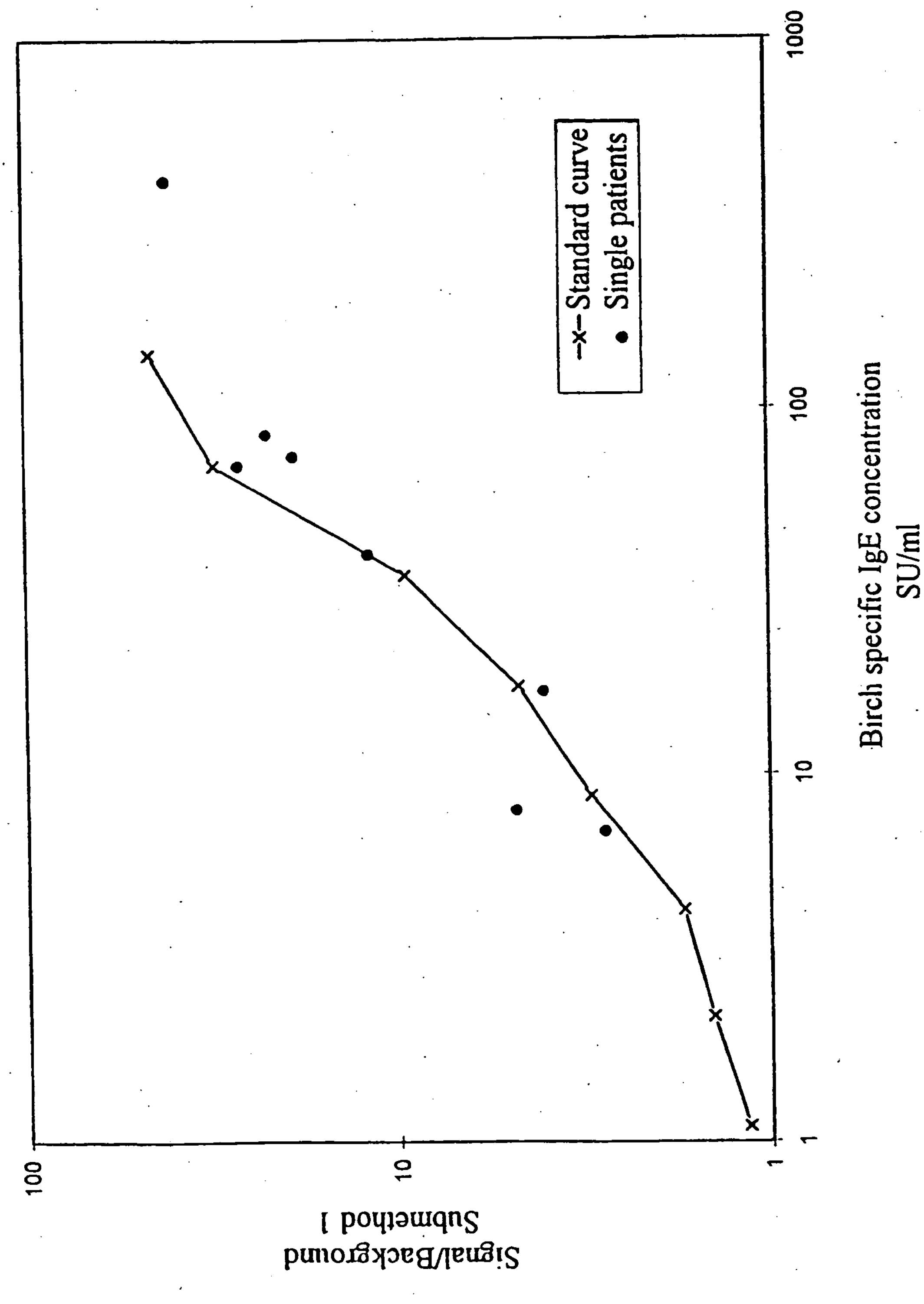

100
10
1
1
10
100
1000
—x— Standard curve
● Single patients
Signal/Background
Submethod 1
Birch specific IgE concentration
SU/ml

Figure 7

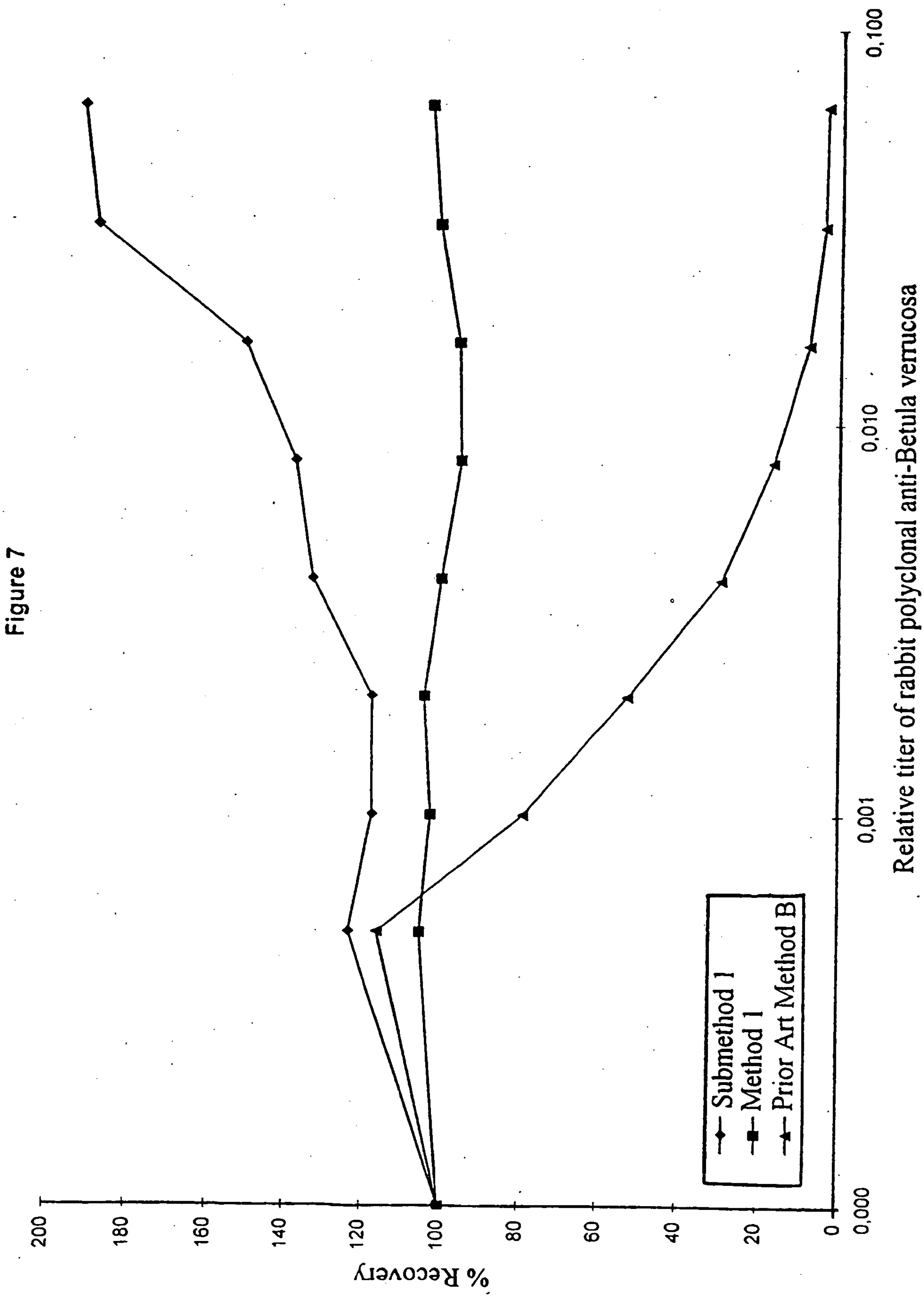
% Recovery
200
180
160
140
120
100
80
60
40
20
0
0,000
0,001
0,010
0,100
Relative titer of rabbit polyclonal anti-Betula verrucosa
Submethod 1
Method 1
Prior Art Method B

Figure 8

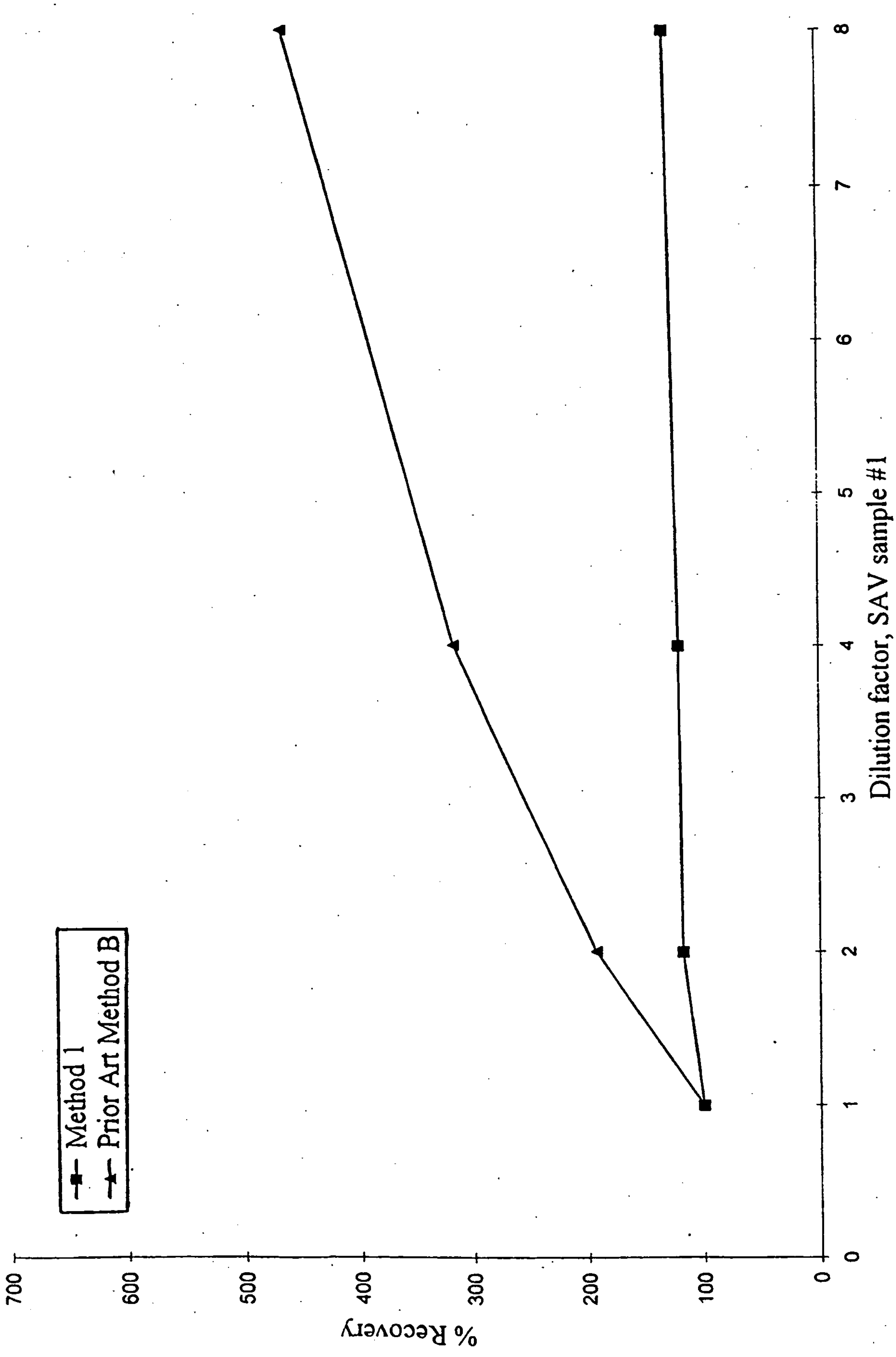
Method 1
Prior Art Method B
% Recovery
0
100
200
300
400
500
600
700
0
1
2
3
4
5
6
7
8
Dilution factor, SAV sample #1

Figure 9

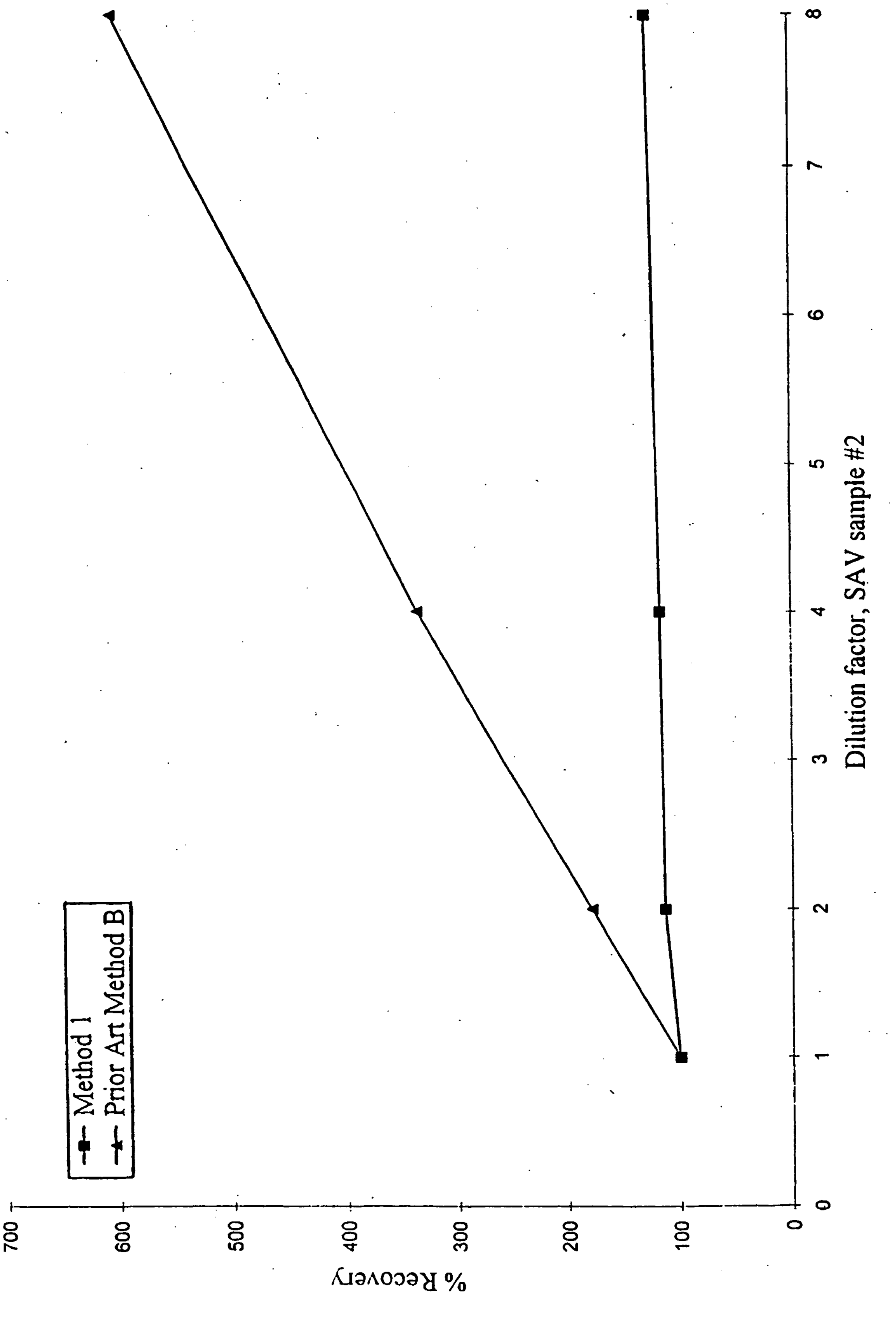
Method 1
Prior Art Method B
% Recovery
0
100
200
300
400
500
600
700
Dilution factor, SAV sample #2
0
1
2
3
4
5
6
7
8

Figure 10

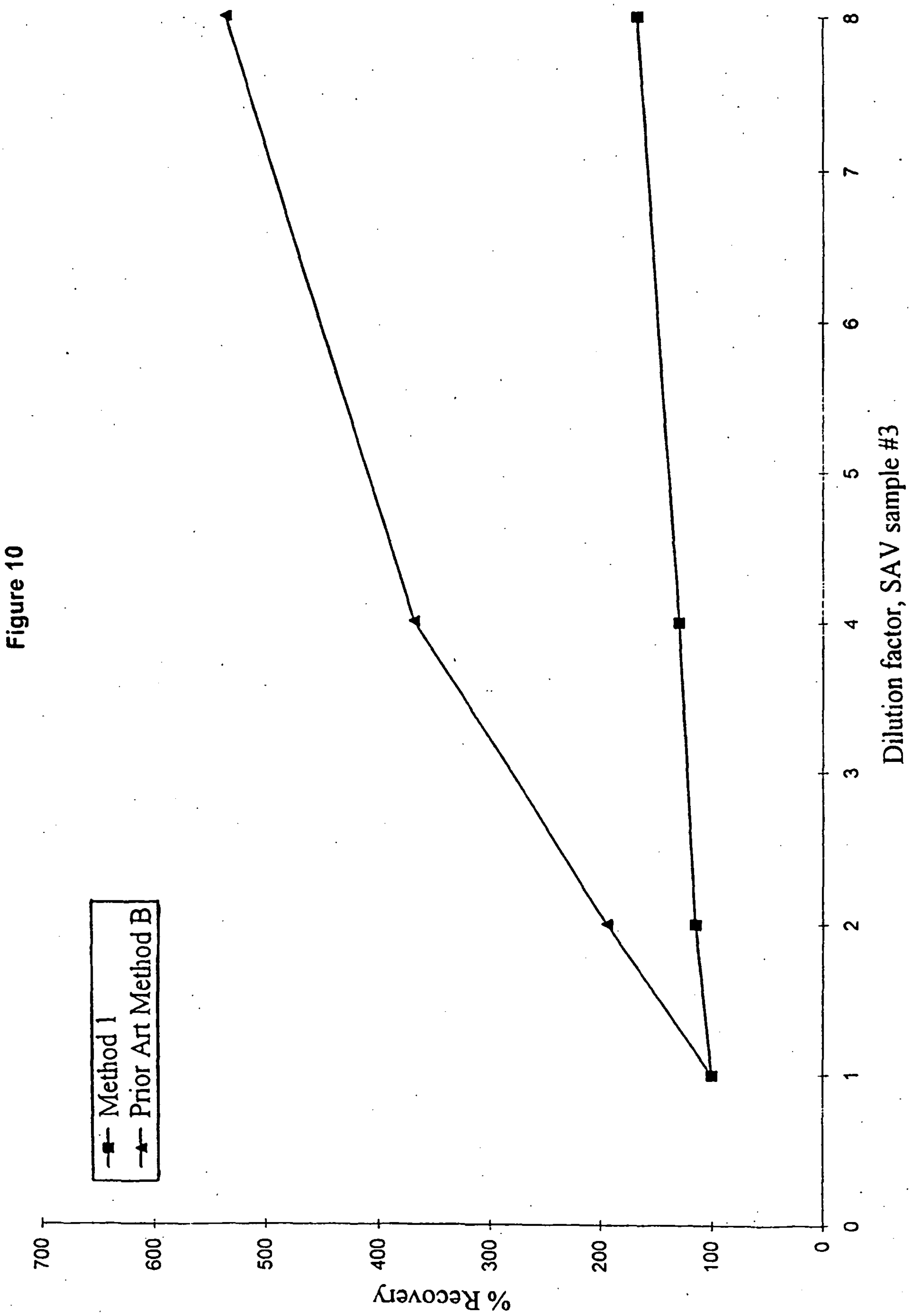
Method 1
Prior Art Method B
% Recovery
700
600
500
400
300
200
100
0
0
1
2
3
4
5
6
7
8
Dilution factor, SAV sample #3

Figure 11

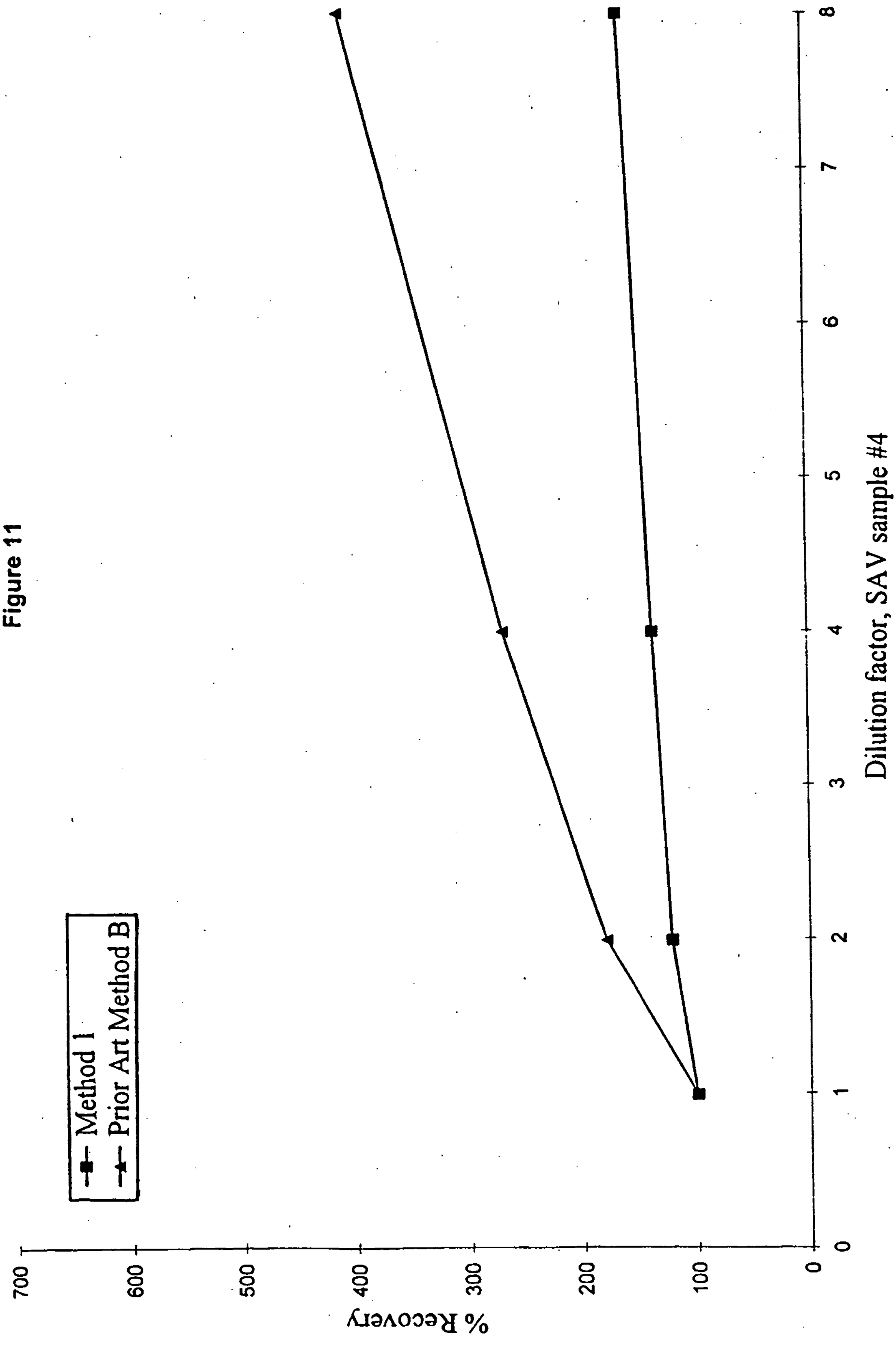
Method 1
Prior Art Method B
% Recovery
700
600
500
400
300
200
100
0
0
1
2
3
4
5
6
7
8
Dilution factor, SAV sample #4

Figure 12

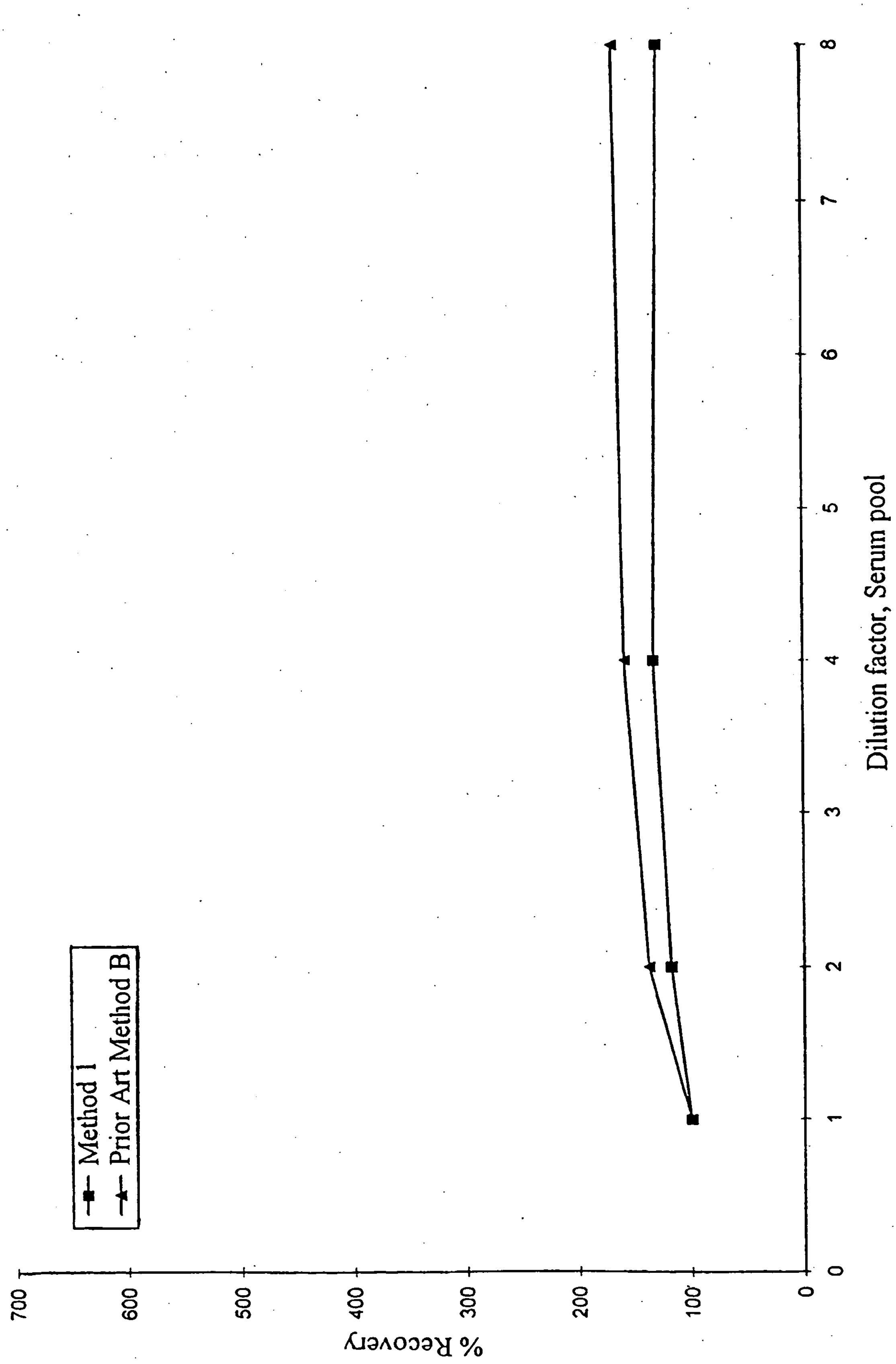
Method 1
Prior Art Method B
% Recovery
0
100
200
300
400
500
600
700
Dilution factor, Serum pool
0
1
2
3
4
5
6
7
8

Figure 13

Specific IgE determined by Prior Art Method B

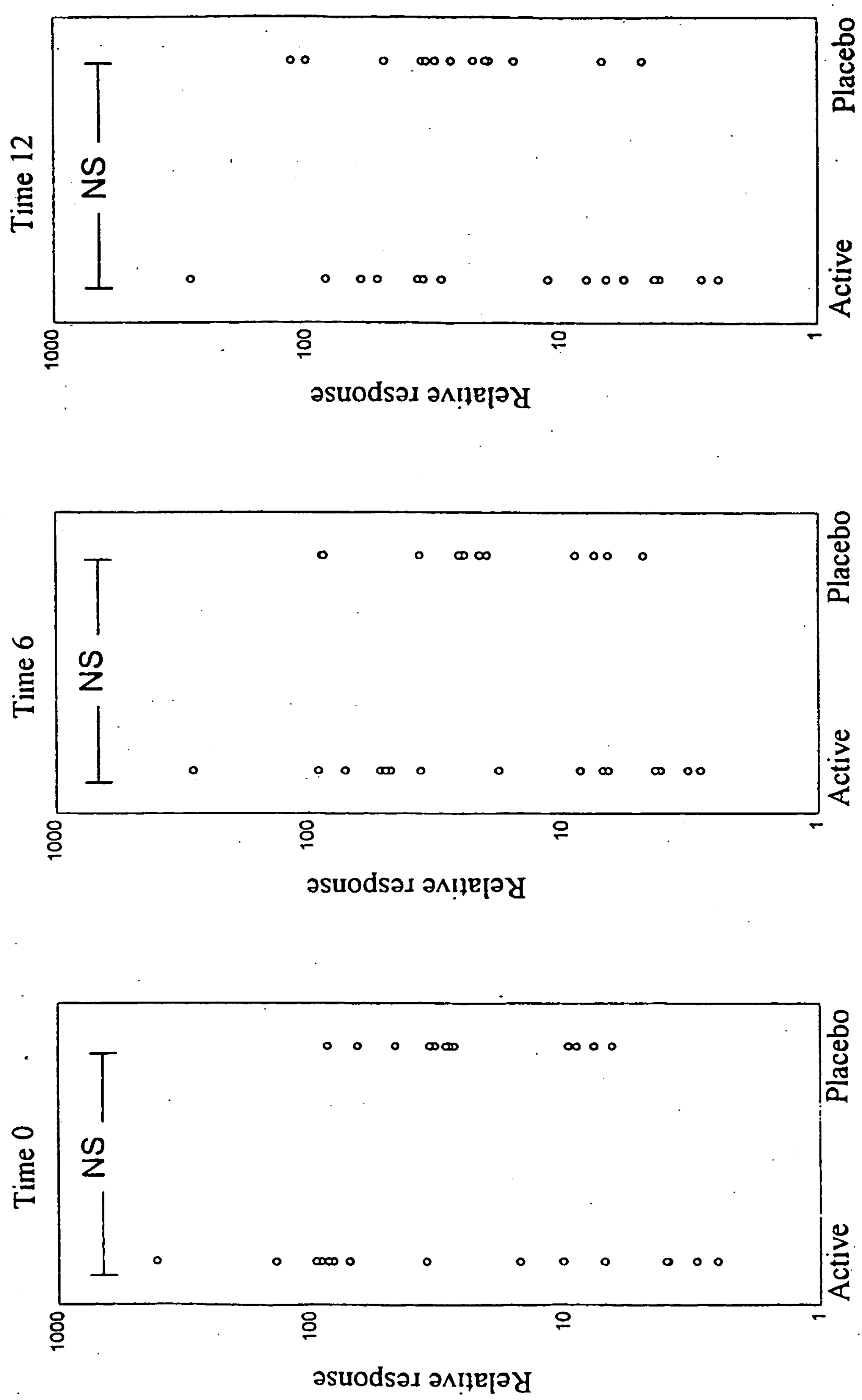

Figure 14

Specific IgE determined by Method 1

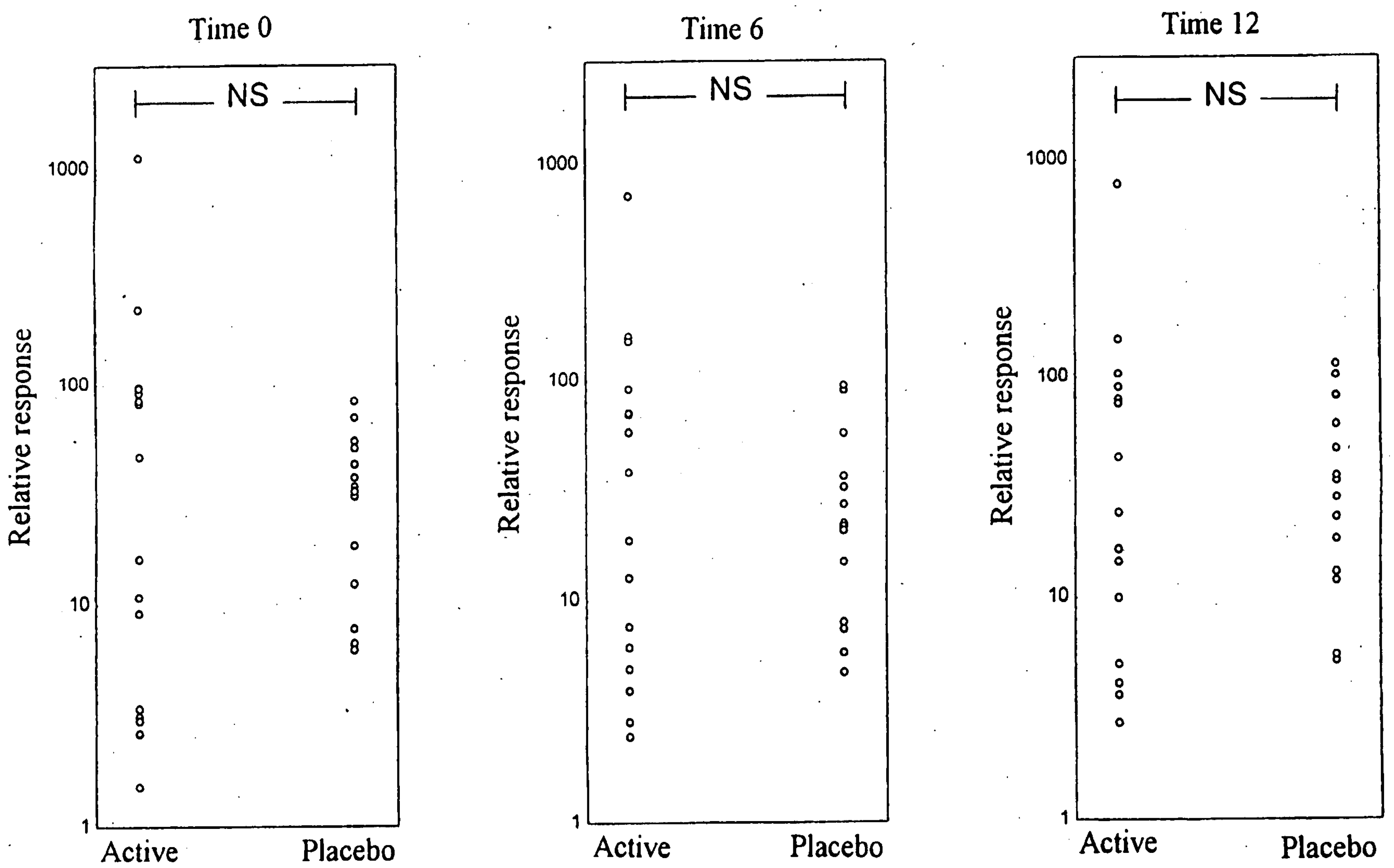

Figure 15 Ratio between specific IgE determined by Prior Art Method B and Method 1

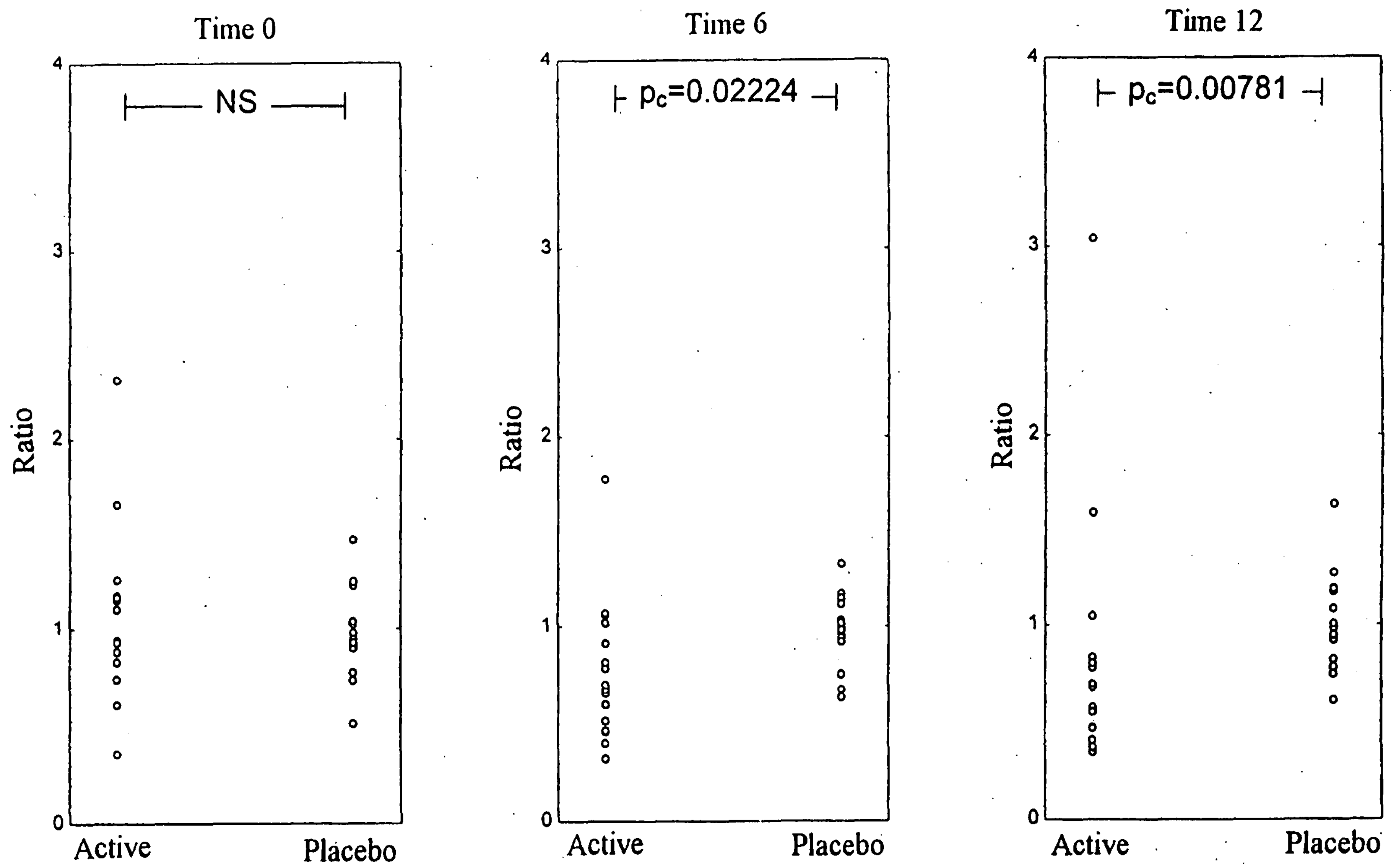

Fig. 16

EFFECT

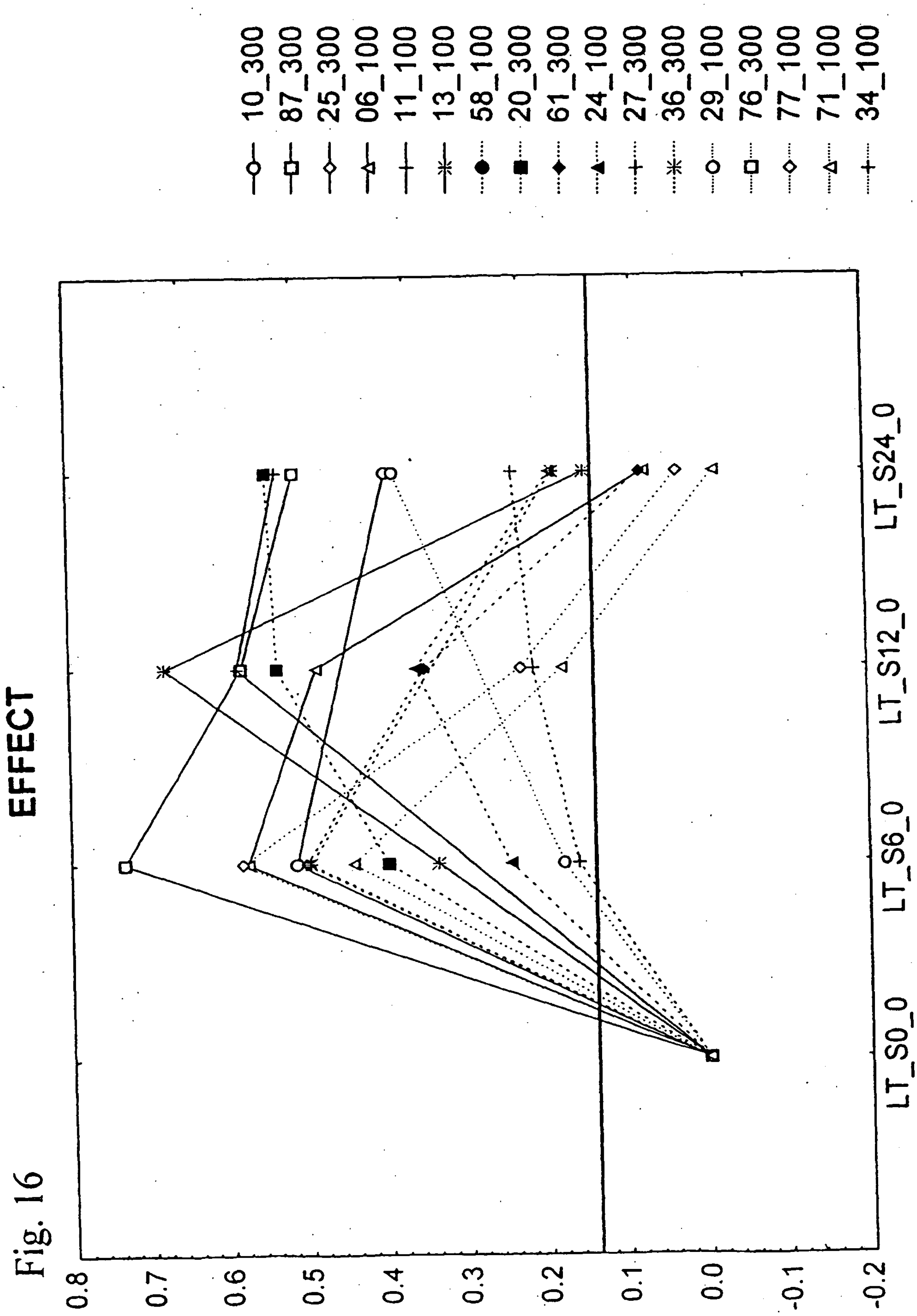

Fig. 17

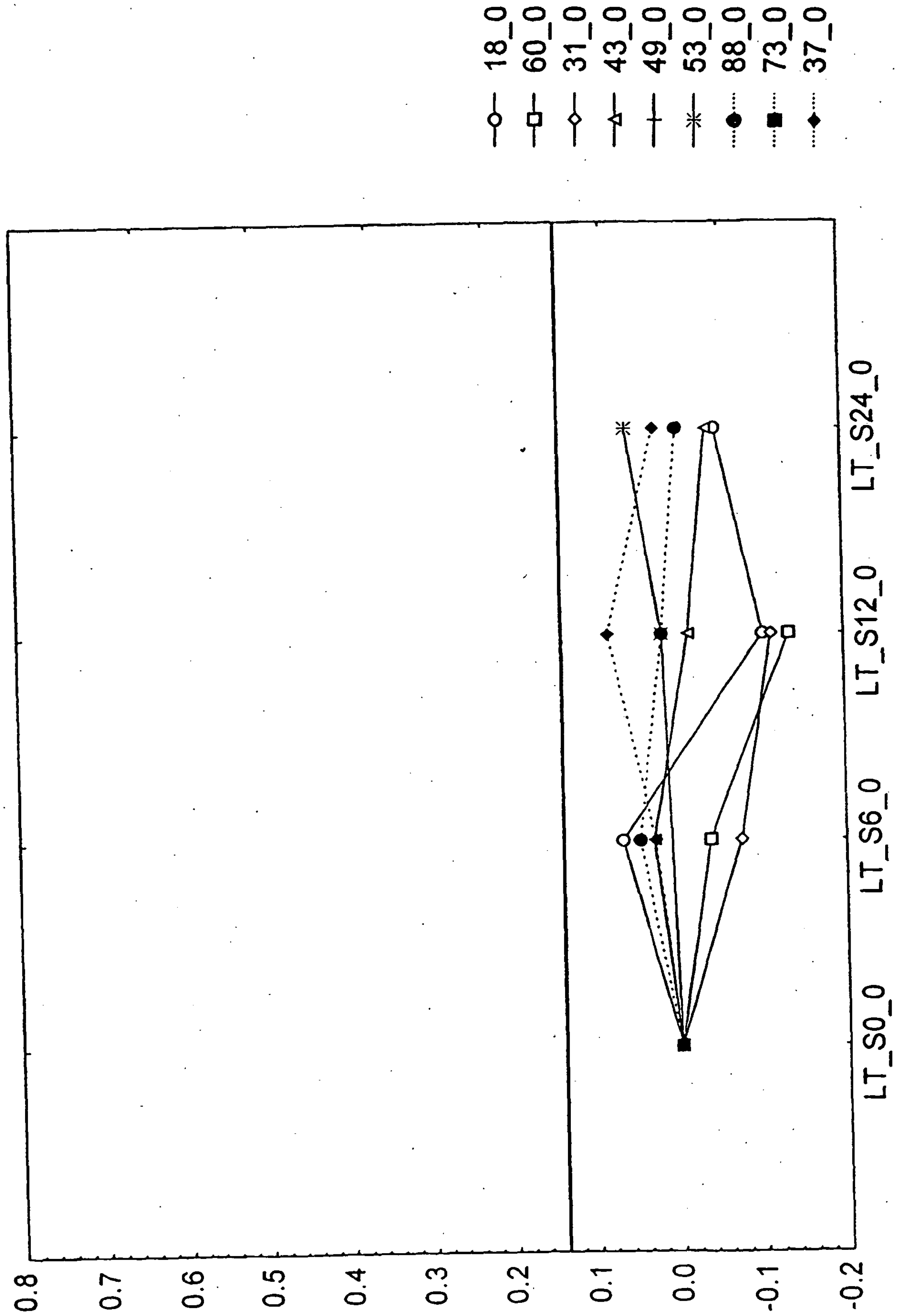
NO EFFECT
18_0
60_0
31_0
43_0
49_0
53_0
88_0
73_0
37_0
0.8
0.7
0.6
0.5
0.4
0.3
0.2
0.1
0.0
-0.1
-0.2
LT_S0_0
LT_S6_0
LT_S12_0
LT_S24_0

Fig. 18

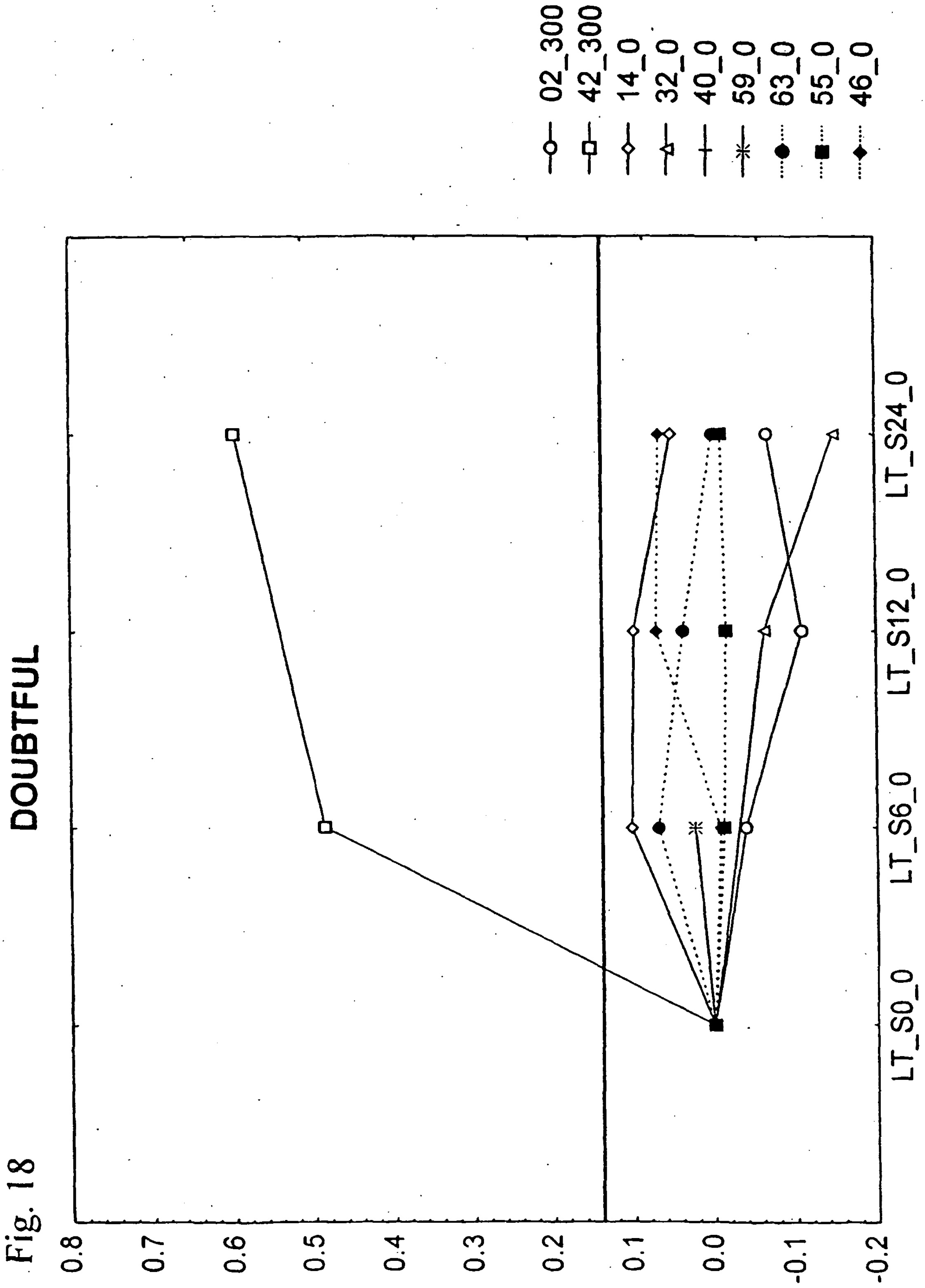
DOUBTFUL
0.8
0.7
0.6
0.5
0.4
0.3
0.2
0.1
0.0
-0.1
-0.2
LT_S0_0
LT_S6_0
LT_S12_0
LT_S24_0
02_300
42_300
14_0
32_0
40_0
59_0
63_0
55_0
46_0

Fig. 19

Dose-response relation of the effect parameter

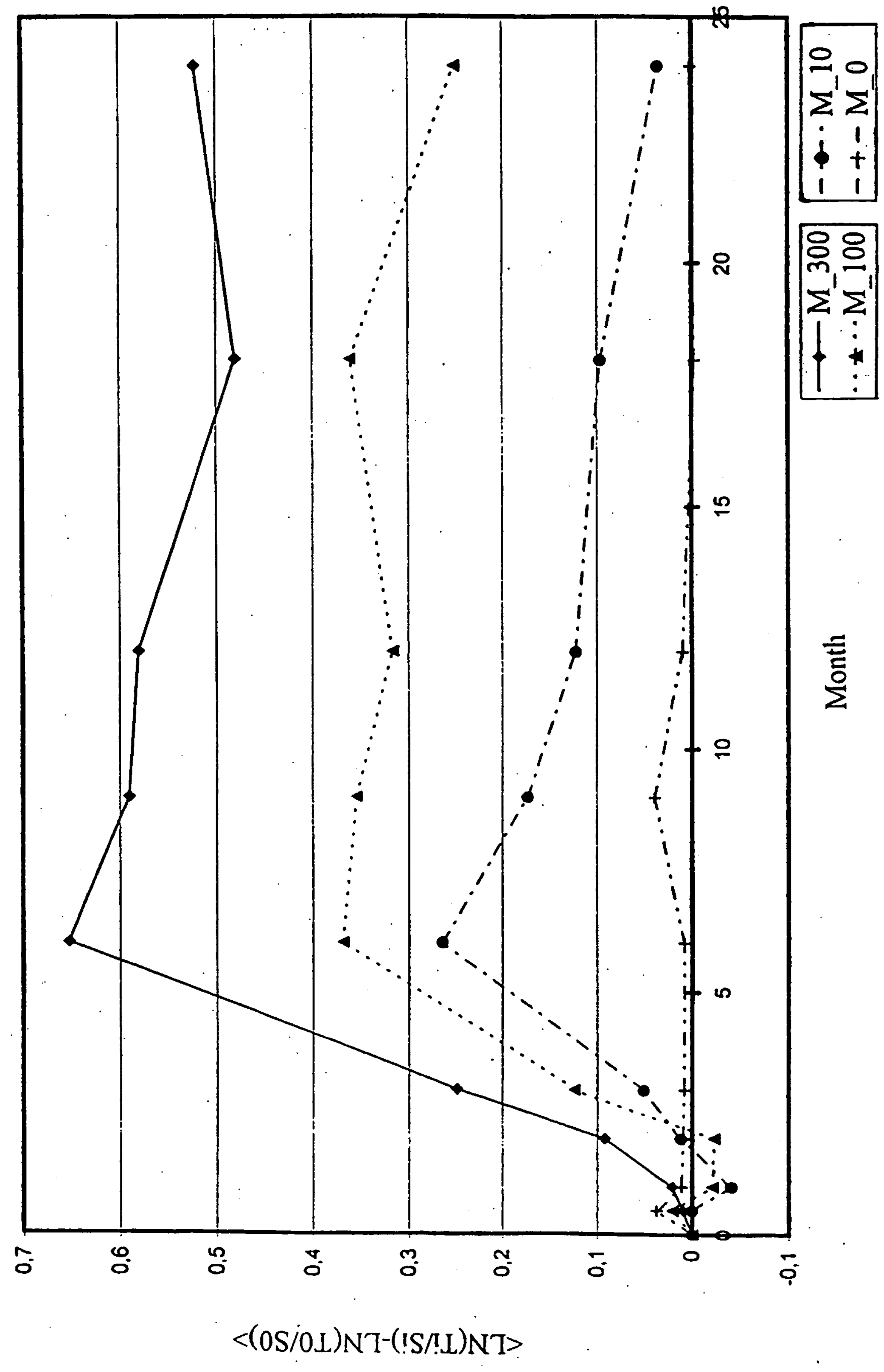

Fig. 20

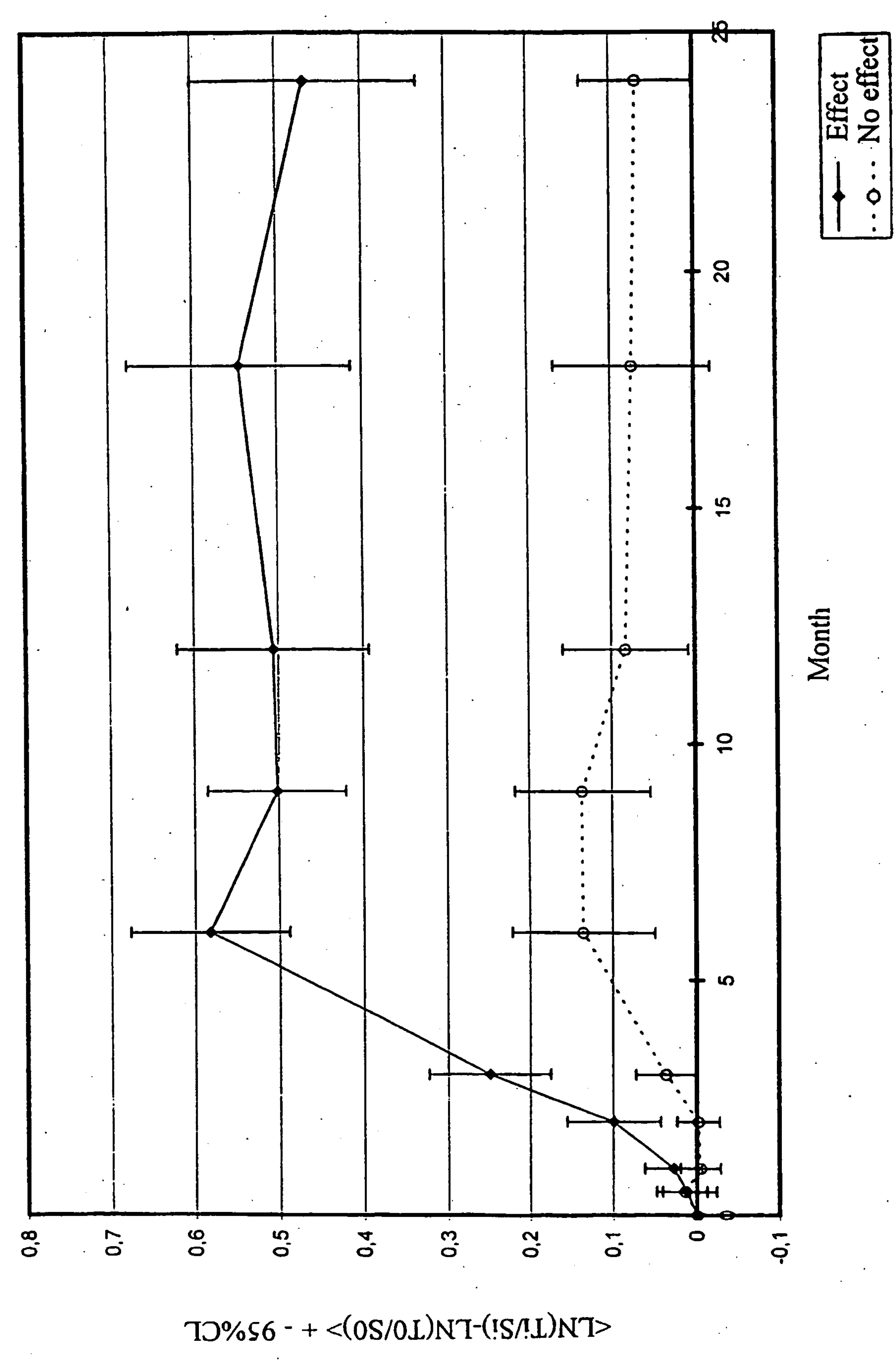
Mean effect
<LN(Ti/Si)-LN(T0/S0)> + - 95%CL
0,8
0,7
0,6
0,5
0,4
0,3
0,2
0,1
0
-0,1
0
5
10
15
20
25
Month
Effect
No effect

Fig. 21

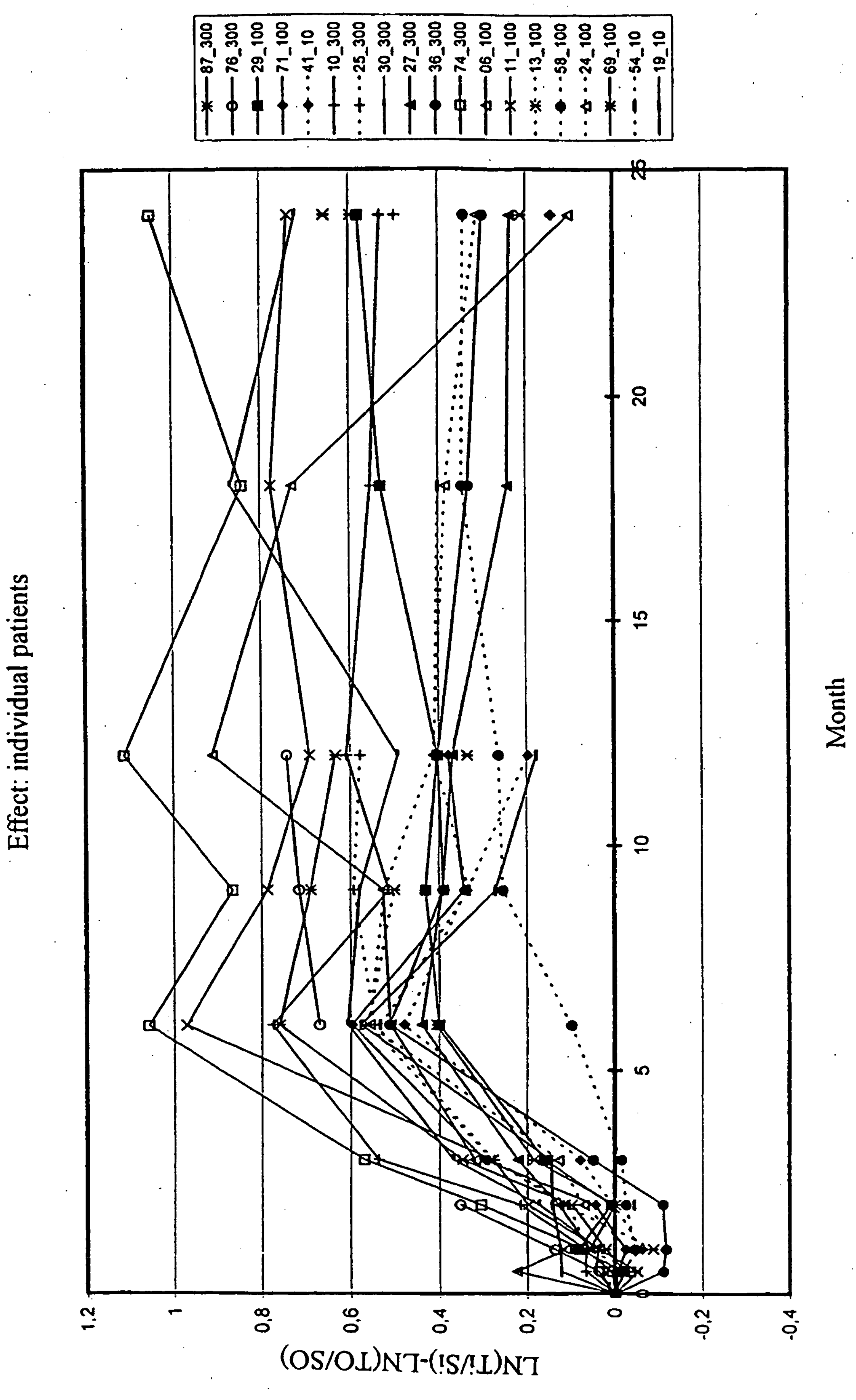
Effect: individual patients
87_300
76_300
29_100
71_100
41_10
10_300
25_300
30_300
27_300
36_300
74_300
06_100
11_100
13_100
58_100
24_100
69_100
54_10
19_10
1,2
1
0,8
0,6
0,4
0,2
0
-0,2
-0,4
LN(Ti/Si)-LN(T0/S0)
5
10
15
20
25
Month

Fig. 22

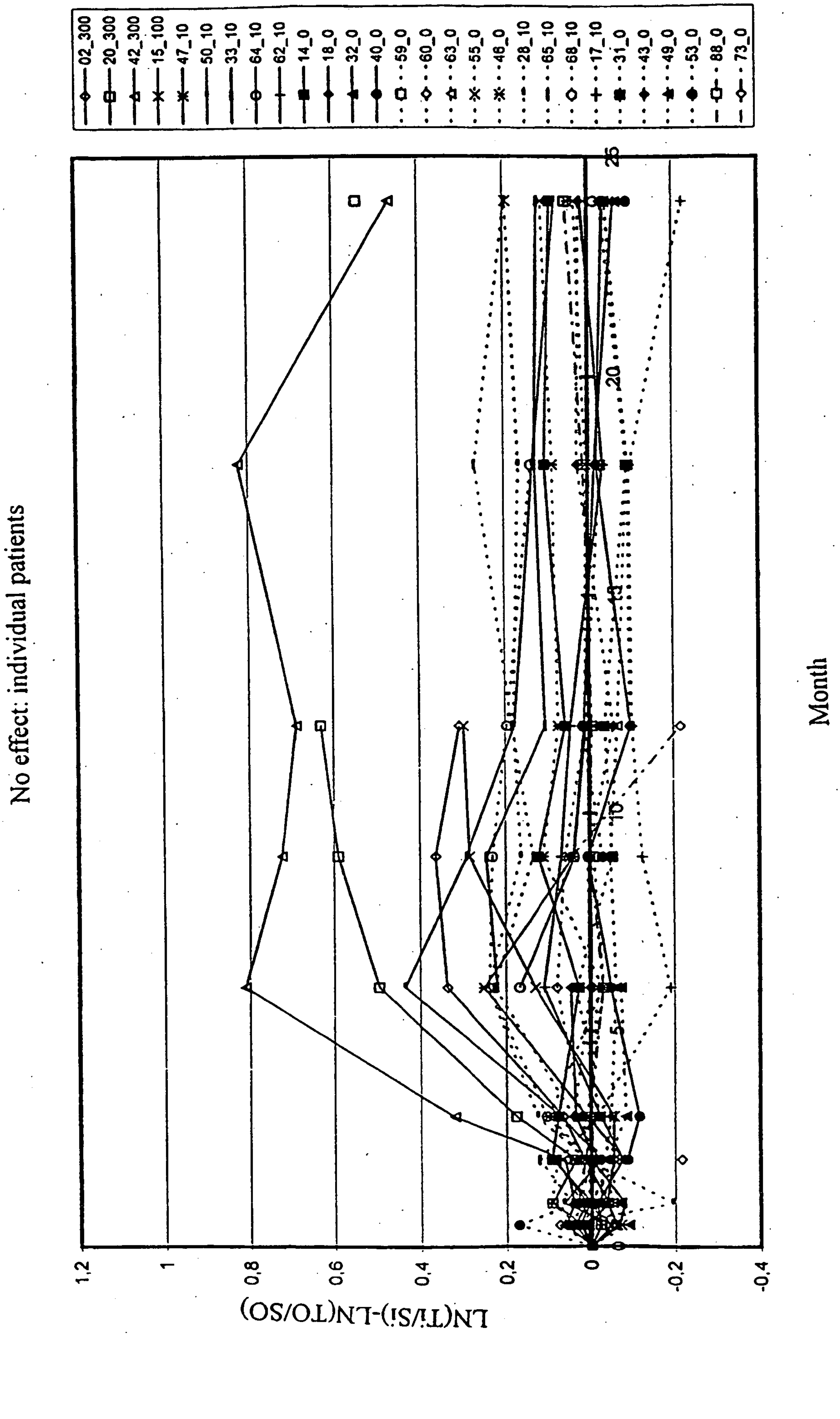
No effect: individual patients
LN(Ti/Si)-LN(TO/SO)
1,2
1
0,8
0,6
0,4
0,2
0
-0,2
-0,4
Month
5
10
15
20
25
02_300
20_300
42_300
15_100
47_10
50_10
33_10
64_10
62_10
14_0
18_0
32_0
40_0
59_0
60_0
63_0
55_0
46_0
28_10
65_10
68_10
17_10
31_0
43_0
49_0
53_0
88_0
73_0

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9411734 A
- WO 9816829 A


### Non-patent literature cited in the description


- **V. OLIVIERI et al.** Capture assay for specific IgE. *Journal of Immunological Methods,* 1993, vol. 157, 65-72
- **WEEKS et al.** *Clinical Chem.,* 1983, vol. 29, 1474-1479
- **IAN WEEKS ; IRAJ BEHESHTI ; FRANK MCCAPRA ; ANTHONY K. CAMPBELL ; J. STUART WOODHEAD.** *Clinical Chemistry,* 1983, vol. 29, 1474-1479
- **J. BOLAND ; G. CAREY ; E. KRODEL ; M. KWIATKOWSKI.** *Clinical Chemistry,* 1990, vol. 36, 1598-1602